# EUROPÄISCHE PATENTSCHRIFT

(11) **EP 4 114 295 B1**
(45) Veröffentlichungstag und Bekanntmachung des Hinweises auf die Patenterteilung: **13.08.2025**
(21) Anmeldenummer: 21707703.1
(22) Anmeldetag: 26.02.2021
(51) Int. Cl.: A61B 17/80, A61B 90/96, A61B 17/82, A61B 17/04, A61B 90/00

(54) **KIT, PLATTE UND INSERT ZUR VERSORGUNG EINER CLAVICULA**
KIT, PLATE AND INSERT FOR SUPPLYING A CLAVICULA
KIT, PLAQUE ET INSERT DE FOURNITURE D'UNE CLAVICULE

(30) Priorität: 03.03.2020 EP 20160793
(43) Veröffentlichungstag der Anmeldung: 11.01.2023
(73) Patentinhaber: Medartis Holding AG, 4057 Basel (CH)
(72) Erfinder: ZEUNER, Hermann, 79111 Freiburg (DE); KOHUT, Georges, 1700 Fribourg (CH); STEIGER, Cornelia, 8805 Richterswil (CH)
(74) Vertreter: Hepp Wenger Ryffel AG
(86) Internationale Anmeldenummer: PCT/EP2021/054887
(87) Internationale Veröffentlichungsnummer: WO 2021/175724

(56) Entgegenhaltungen:
- EP-A2- 1 836 981
- WO-A1-2019/108222
- DE-A1- 10 039 767
- US-A1- 2008 027 439
- US-A1- 2019 321 182

## Beschreibung

Die Erfindung betrifft ein Kit, eine Platte und ein Insert zur Behandlung eines Knochens, insbesondere einer Clavicula, mit den Merkmalen des Oberbegriffes der unabhängigen Ansprüche.

Es ist bekannt, Knochen, beispielsweise die menschliche Clavicula, insbesondere bei Frakturen mit implantierten Platten zu versorgen.

US 2007/0185493 offenbart eine Clavicula-Platte mit einem abstehenden Flügel zur Befestigung einer Schraube.

US 2005/085819 offenbart mehrstückige Implantate zur Behandlung von Knochenbrüchen.

US 9,149,312 offenbart eine Clavicula-Platte, bei der zusätzlich mittels eines Fadens ein weiteres Körperteil mit der Platte verbunden werden kann.

US 2008/027439 A1 offenbart ein Halswirbelsäulenplattensystem mit einem ersten Drehelement, das ein Gewindeloch aufweist. Sie offenbart die Merkmale des Oberbegriffs des unabhängigen Anspruchs 1.

Die bekannten Implantate und Methoden weisen aber verschiedene Nachteile auf. So ist es beispielsweise schwierig, die zu implantierende Platte temporär zu fixieren. Weiter ist die Verbindung der Platte mit einem Faden oftmals schwierig. Die Verbindung der Platte mit dem Knochen mittels Knochenschrauben ist ausserdem wenig flexibel.

Es ist daher Aufgabe der vorliegenden Erfindung, die Nachteile des Bekannten zu vermeiden, und ein Kit, eine Platte, und ein Insert bereitzustellen, das einfach implantiert werden kann und daher günstig anzuwenden und wenig fehleranfällig ist, und gleichzeitig einfach an die Anatomie eines Patienten angepasst werden kann.

Erfindungsgemäss wird diese Aufgabe mit einem Kit, einer Platte und einem Insert mit den Merkmalen des unabhängigen Anspruchs 1 gelöst

Vorteilhafte Weiterbildungen der Erfindung sind Gegenstand der abhängigen Ansprüche 2-10.

Der in der vorliegenden Beschreibung verwendete Begriff "Ausführungsform" bzw. Ausführungsbeispiel gibt nicht unbedingt Möglichkeiten zur Ausführung der beanspruchten Erfindung an, sondern enthält auch Beispiele, die zum Verständnis der Erfindung beitragen.

Das erfindungsgemässe Kit umfasst mindestens ein Insert und eine Platte zur Versorgung eines Knochens, insbesondere einer Clavicula. Die Platte weist eine Aufnahme für das Insert auf. Das Insert weist eine Aussenkontur auf und die Aufnahme weist eine Innenkontur auf. Die Innenkontur und die Aussenkontur sind dabei so aufeinander abgestimmt, dass das in die Aufnahme eingesetzte Insert um eine Achse verschwenkbar ist. Die Achse verläuft dabei im Wesentlichen parallel zu einer Plattenoberseite.

Insbesondere kann das Insert benutzt werden, um eine Verbindung mit einem Knochen oder anderem Körperteil herzustellen. Dies kann mit diversen Verbindungsmechanismen erreicht werden, wie untenstehend im Detail ausgeführt wird. Besonders vorteilhaft ist die Verbindung mit einer Schraube oder einem Faden. Da das Insert im eingesetzten Zustand verschwenkbar ist, ist die Verankerung im Allgemeinen flexibler, da sie auch während der Implantation angepasst werden kann, zum Beispiel in ihrer Orientierung.

Das Insert ist derart ausgebildet, dass es in der definitiven Einsetzposition weiterhin in der Aufnahme schwenkbar bleibt, wenn eine Schraube oder ein Faden eingesetzt ist.

Das Insert in der Aufnahme um genau eine Achse parallel zu einer Plattenoberseite verschwenkbar.

Bevorzugt ist die Aussenkontur des Inserts und/oder die Innenkontur der Aufnahme zumindest teilweise gerundet. Zusätzlich oder alternativ können die Aussenkontur und/oder die Innenkontur eine mindestens teilweise rotationssymmetrische Form, besonders bevorzugt mindestens teilweise eine kreiszylindrische, gestuft zylindrische, konische, elliptische, hyperbolische oder parabolische Form, insbesondere in einem Querschnitt senkrecht zu einer Längsachse, aufweisen.

Eine zumindest teilweise, bevorzugt vollständig, rotationssymmetrische Struktur von Innenkontur und/oder Aussenkontur senkrecht zu einer Längsachse ist vorteilhaft zur genau definierten Anbringung des Inserts, welche gleichzeitig eine genau definierte Verschwenkung erlaubt. Somit kann das Insert schnell und zuverlässig eingesetzt werden, da die Oberseite und Unterseite des Inserts sich vorzugsweise bei einem solchen rotationsymmetrischen Inserts nicht unterscheiden. Somit kann das Insert nicht seitenverkehrt in der Aufnahme angebracht werden. Alternativ können sich Ober- und Unterseite des Inserts auch unterscheiden, d.h. es ist nur eine Richtung der korrekten Abringung möglich.

Zudem ist das Insert vorzugsweise so ausgebildet, dass das Insert vollständig in der Aufnahme der Platte angeordnet werden kann.

Somit kann das Insert bündig und/oder abgesenkt zur Platte in der Aufnahme angeordnet sein. Somit weist die Platte durch das Insert keine Unebenheiten auf.

Zudem erlaubt es diese Anordnung, die Platte bündig zum Knochen anzuordnen, unabhängig von der Positionierung des Inserts. Somit kann eine Fixierung, insbesondere eine temporäre Fixierung, leichter vorgenommen werden.

Eine teilweise gerundete Form soll insbesondere Formen umfassen, die einen kreisförmigen Querschnitt, beispielsweise entlang einer Längsachse aufweisen. Ebenso sollen alle Formen, die entlang einer Oberfläche ohne Kanten und nicht gerade ausgeführt sind, als gerundet verstanden werden.

Solche Formen ermöglichen auf besonders einfache Weise eine Verschwenkbarkeit des Inserts in der Aufnahme.

Bevorzugt sind die Aussenkontur und die Innenkontur so aufeinander abgestimmt, dass das in die Aufnahme eingesetzte Insert in einer Längsrichtung der Platte verschiebbar ist. Die Verschiebung ist insbesondere so, dass das Insert sich entlang der Längsrichtung in verschiedenen Positionen stabil einsetzen lässt. Dazu weist die Aufnahme insbesondere in Längsrichtung eine Auflagefläche auf, die parallel zu Plattenunterseite verläuft und die länger ist, als die Ausdehnung des Inserts in der Längsrichtung.

Dadurch wird eine weitere Anpassung der Verankerung der Platte und des Inserts an einem Körperteil, beispielsweise einem Knochen ermöglicht.

Die Verschiebbarkeit erlaubt es, eine Verankerung flexibel der Form eines Körperteils, wie einem Knochen, anzupassen. Somit kann die Grösse oder Biegung eines Knochens, wie beispielsweise der Clavicula, bei der Verankerung berücksichtigt werden. Zudem kann eine flexible Anbringung einer Verankerung Spannungen zwischen Verankerungen vermeiden.

Das Insert ist zur Fixierung eines Fadens und bevorzugt zur Aufnahme einer Schraube angepasst.

Gemäss einem ersten erfindungsgemässen Aspekt weist das Insert bevorzugt mindestens einen Steg auf, der zum Halten eines Fadens angepasst ist. Besonders bevorzugt umfasst das Insert genau einen Steg.

Dies ermöglicht es, einen Faden auf besonders einfache Weise, beispielsweise durch einen Knoten oder eine Schlinge, am Insert zu befestigen. Insbesondere kann ein Steg auch innerhalb einer durchgehenden Öffnung angeordnet sein, so dass ein Faden durch das Insert hindurch angeordnet werden kann. Insbesondere kann dadurch auch die Knochenplatte an einem Körperteil verankert werden.

Bevorzugt ist der Steg versenkt. Versenkt soll hierbei so verstanden werden, dass der Steg innerhalb der Öffnung so angeordnet ist, dass er zumindest auf einer Seite nicht bündig mit der Oberfläche des Inserts ist. Daher ist ein versenkter Steg in der Regel dünner ausgeführt als das Insert. Er kann aber auf einer Seite bündig an die Oberfläche des Inserts angeordnet sein. Alternativ kann er so innerhalb der Öffnung angeordnet sein, dass er von beiden Seiten der Öffnung betrachtet versenkt ist, also mit keiner Oberfläche des Inserts bündig angeordnet ist.

Die durchgehende Öffnung ist vorzugsweise so auf dem Insert angeordnet, dass bei ordnungsgemässer Anbringung des Inserts in der Aufnahme, ein Durchlass zur Aufnahme eines Fadens oder einer Schraube von der Plattenoberseite zur Plattenunterseite entsteht.

Die Öffnung des Inserts in der Aufnahme ist bevorzugt auf der Plattenoberseite anordenbar.

Somit kann das Insert durch eine Schraube und/oder einen Faden leicht fixiert werden.

Dadurch kann insbesondere auch ein Knoten im Insert versenkt sein, was zu einer Reduktion von Irritationen des Gewebes führen kann.

Das Kit umfasst einen Faden zum Fixieren eines Körperteils. Besonders bevorzugt ist der Faden zum Fixieren eines Coracoids angepasst.

Alternativ umfasst das Insert gemäss einem zweiten erfindungsgemässen Aspekt eine Öffnung zur Aufnahme einer Schraube, insbesondere einer Knochenschraube.

Die Öffnung kann insbesondere mit einem Gewinde ausgeführt sein. Es ist aber auch möglich, eine gewindelose Öffnung zu verwenden. Insbesondere kann es sich bei der Öffnung um eine Bohrung handeln, die im Wesentlichen zylinderförmig und mit einem kreisrunden Querschnitt ausgebildet ist. Alternativ sind auch andere Querschnitte der Öffnung denkbar, z. B. quadratisch, elliptisch, dreieckig oder rechteckig. Der Querschnitt entlang einer Achse senkrecht zur Achse der Bohrung ebenfalls rund, elliptisch, rechteckig, quadratisch oder rechteckig sein, insbesondere wenn die Öffnung als Langloch ausgestaltet ist. Es ist ebenfalls denkbar, die Öffnung als Verblockungsloch auszugestalten.

Dadurch lässt sich das Insert mit der Schraube an einem Knochen befestigen. Dies ermöglicht eine weitere Verankerung der Platte an einem Knochen. Durch die Verschwenkbarkeit des Inserts kann insbesondere auch der Eintrittswinkel der Schraube in den Knochen angepasst werden.

Bevorzugt umfasst das Kit mindestens eine Schraube, insbesondere eine nicht verblockbare Schraube. Alternativ kann die Schraube auch verblockbar sein. Besonders bevorzugt ist die Schraube derart an die Öffnung des Inserts angepasst, dass sie durch diese Öffnung hindurch eingeschraubt werden kann. Insbesondere kann der Schraubendurchmesser an einen Innendurchmesser der Öffnung des Inserts und/oder das Aussengewinde der Schraube an ein Innengewinde oder eine Verblockungskontur in der Öffnung des Inserts angepasst sein.

Die Schraube kann durch das Innengewinde oder die Verblockungskontur in der Öffnung des Inserts hindurch eingeschraubt werden und das Aussengewinde der Schraube in den Knochen eingeschraubt werden. Eine Verblockungskontur am Kopf der Schraube verankert sich dann in der Gegenkontur des Inserts. Somit kann die Schraube fest mit dem Insert verblockt werden und gleichzeitig am Knochen fixiert werden.

Die Schraube kann auch einen Schraubenkopf umfassen, der in seiner Grösse so angepasst ist, dass er nicht durch die Öffnung passt. Bei entsprechender Ausführung der Öffnung kann der Schraubenkopf auch so geformt und in seiner Grösse angepasst sein, dass er in der Öffnung des Inserts versenkt werden kann. Es ist ebenfalls denkbar, den Schraubenkopf als Verblockungskopf auszubilden.

Bevorzugt weist das Insert eine Halterung auf. Das Insert und die Halterung sind dabei über eine Sollbruchstelle miteinander verbunden.

Dadurch kann das Insert einfach gehalten werden und in der gewünschten Position in eine Aufnahme eingebracht werden. Durch die Sollbruchstelle kann zu einem gewünschten Zeitpunkt, z.B. nach Einbringen in die Aufnahme, das Insert von der Halterung entfernt werden. Die Halterung kann auch so ausgeführt sein, dass sie neben dem Halten auch zum gleichzeitigen Einfädeln des Fadens dient.

Bevorzugt weist die Platte auf ihrer Oberseite mindestens eine die Platte nicht durchdringende Senkung auf. Diese ist zur temporären Aufnahme eines Werkzeuges, bevorzugt einer Zange, angepasst.

Bevorzugt umfasst das Kit ein Werkzeug, besonders bevorzugt eine Zange. Das Werkzeug umfasst dabei mindestens eine Spitze, deren Form und Grösse so angepasst ist, dass sie mit der Senkung der Platte in Wirkverbindung gebracht werden kann.

Die Erfindung bezieht sich weiterhin auf ein Insert. Das Insert ist besonders zur Verwendung in einem Kit wie oben beschrieben geeignet. Der Fachmann versteht daher, dass sämtliche hier beschriebenen Eigenschaften auch im Kit zur Anwendung kommen können. Ebenso können alle vorgängig beschriebenen Eigenschaften des Inserts als Teil des Kits zusätzlich oder alternativ auch für ein einzelnes Insert verwendet werden. Das Insert ist zur Aufnahme in eine Knochenplatte angepasst und umfasst mindestens eine Öffnung. Die Öffnung ist zur Aufnahme einer Schraube und/oder zur Fixierung eines Fadens angepasst. Das Insert ist zudem über eine Sollbruchstelle mit einer Halterung verbunden.

Das Insert ist besonders bevorzugt teilweise gerundet ausgestaltet. Es ist auch möglich, ein Insert mit einem rotationssymmetrischen Querschnitt senkrecht zur Längsachse auszugestalten. Es wäre auch denkbar, dass der Querschnitt des Inserts eine n-zählige Drehsymmetrie aufweist, wobei n eine beliebige ganze Zahl sein grösser als 2, besonders bevorzugt mindestens 3, sein kann. So ist beispielsweise auch ein rechteckiger Querschnitt denkbar.

Bevorzugt umfasst das Insert einen Steg, der innerhalb der Öffnung angeordnet ist und insbesondere darin versenkt ist. Bevorzugt ist die mindestens eine Öffnung zur Aufnahme einer Schraube angepasst. Bevorzugt beträgt der Gewindedurchmesser der Schraube 1 bis 8 mm, besonders bevorzugt 2 bis 5 mm, noch bevorzugter 2.5 bis 3.5 mm.

Es wird hierin auch beschrieben aber nicht beansprucht, eine Platte zur Behandlung eines Knochens, insbesondere einer Clavicula. Die Platte ist besonders geeignet zur Verwendung in einem Kit wie vorgängig beschrieben. Es ist für den Fachmann auch klar, dass sämtliche vorgängig, im Zusammenhang mit dem Kit beschriebenen Eigenschaften einer Platte auch für diese Platte eingesetzt werden können. Die Platte umfasst mindestens ein Schraubenloch zur Aufnahme einer Knochenschraube. Die Platte weist in einem Endbereich wenigstens eine abstehende Lasche auf.

Die Lasche ist insbesondere geeignet zur Anformung an die individuelle Anatomie und zur Fixierung an einen Knochen oder anderem Körperteil. Sie ermöglicht daher die Fixierung aus verschiedenen Winkeln oder Positionen heraus, was die Behandlung flexibler macht.

Bevorzugt umfasst die Platte mindestens eine, besonders bevorzugt mindestens zwei, noch bevorzugter genau zwei, Laschen. Bevorzugt ist die mindestens eine Lasche über mindestens eine biegbare Brücke mit der Platte verbunden. Die Lasche weist insbesondere eine Aufnahmeöffnung für eine Knochenschraube auf.

Dies ermöglicht, die Platte mittels einer Knochenschraube an einem Knochen zu befestigen. Insbesondere kann die Brücke in verschiedene Positionen und/oder Orientierungen an den Knochen angepasst werden, so dass eine Schraube entsprechend in verschiedenen Positionen und/oder Orientierungen in den Knochen eingebracht werden. So ist eine besonders vorteilhafte Behandlung möglich, die bei Bedarf gezielt an die Anatomie des Patienten angepasst werden kann.

Bei sehr schlechter Knochenstruktur besteht die Gefahr von Schraubenausrissen. Mittels der o.g. Laschen können Schrauben zusätzlich aus anderen Richtungen gesetzt werden und damit diese Schraubenausrisse verhindern.

Es ist auch denkbar, dass eine Lasche mit zwei oder mehr Brücken mit der Platte verbunden ist. Beispielsweise kann eine Lasche über eine Doppelbrücke mit der Platte verbunden sein.

Der Begriff biegbar soll zumindest ausreichende Duktilität des Materials umfassen, so dass das Material plastisch verformbar ist, ohne zu brechen. Insbesondere soll biegbar auch so verstanden werden, dass eine plastische Verformung mittels Handwerkzeugen oder von blosser Hand erreicht werden kann, beispielsweise durch geeignete Materialauswahl oder Dimensionierung des entsprechenden Teils.

Bevorzugt weist die Platte im Bereich des Schraubenloches oder der Aufnahme eine grössere Plattendicke auf als im Bereich der Brücke.

Bevorzugt ist die Lasche und/oder die Brücke derart geformt, dass ein Winkel zwischen einer Längsachse der Aufnahmeöffnung für eine Knochenschraube und einer Normale an die Oberfläche der Platte höchstens 150° beträgt. Besonders bevorzugt beträgt der Winkel höchstens 120°, noch bevorzugter höchstens 90°.

Es wird hierin auch beschrieben aber nicht beansprucht, eine Platte zur Versorgung eines Knochens, insbesondere der Clavicula. Die Platte ist besonders geeignet zur Verwendung in einem Kit wie vorgängig beschrieben. Es ist für den Fachmann auch klar, dass sämtliche vorgängig im Zusammenhang mit dem Kit beschriebenen Eigenschaften einer Platte auch für diese Platte eingesetzt werden können. Ebenso sind alle vorgängig beschriebenen Merkmale im Zusammenhang mit einer Lasche mit der hier beschriebenen Platte kombinierbar. Die Platte weist mindestens ein Schraubenloch zur Aufnahme einer Knochenschraube auf. Die Platte hat weiter eine Oberseite und eine Unterseite, wobei die Unterseite der Platte in ihrer Form angepasst ist, um auf einem Knochen zumindest teilweise aufzuliegen. Die Oberseite der Platte weist mindestens eine die Platte nicht durchdringende Senkung auf, die zur temporären Aufnahme eines Werkzeuges angepasst ist. Typischerweise handelt es sich beim Werkzeug um ein dem Fachmann bekanntes chirurgisches Instrument.

Eine temporäre Aufnahme dient dazu, einen Form- und/oder Kraftschluss mit einem Werkzeug zu erzeugen, der temporär eine Verschiebung des Werkzeuges relativ zur Knochenplatte ver- bzw. behindert.

Die Platte kann daher auf besonders einfache Weise mittels eines Werkzeuges gehalten werden, was die Behandlung einfacher und sicherer macht, da der Operateur ein Verschieben der Platte relativ zum Knochen verhindern kann. Insbesondere kann die Platte gehalten werden, ohne ein Schraubenloch zu blockieren, weil ein Werkzeug darin eingebracht ist. Ausserdem ermöglicht das Halten in einer dafür angepassten Senkung, die Platte ohne Spiel zu halten. Dem gegenüber ist eine Platte, die mit einem Werkzeug in einem Schraubenloch gehalten wird, etwas bewegbar, wodurch die Behandlung weniger sicher und für den Operateur mühsam sein kann.

Bevorzugt ist die mindestens eine Senkung bezogen auf eine Längsrichtung der Platte, besonders bevorzugt mittig, zwischen Schraubenlöchern angeordnet.

Dadurch kann die Platte, falls gewünscht, z.B. benachbart zu einer Stelle gehalten werden, wo eine Schraube eingeführt werden soll. Dies minimisiert die Abrutschgefahr an der Stelle des verwendeten Schraubenloches und macht die Behandlung sicherer.

Bevorzugt ist die mindestens eine Senkung auf der Plattenoberfläche in einer Querrichtung senkrecht zur Längsrichtung gesehen im Wesentlichen mittig zu einer äusseren Begrenzung der Platte angeordnet.

Dadurch wird die Platte, wenn sie mittels eines in die Senkung eingebrachten Werkzeuges gehalten wird, in der Nähe ihres Schwerpunktes gehalten. Dies reduziert das Risiko eines Verrutschens und macht die Behandlung sicherer.

Es sind selbstverständlich aber auch Senkungen denkbar, die nahe dem Plattenrand oder nahe einem Plattenloch liegen.

Bevorzugt weist die Senkung eine Längsachse auf und ist in Bezug auf die Längsachse im Wesentlichen rotationssymmetrisch ausgebildet.

Rotationssymmetrie soll insbesondere auch n-zählige Drehsymmetrien umfassen, wobei n eine beliebige ganze Zahl sein kann, bevorzugt eine ganze Zahl die mindestens 2, besonders bevorzugt mindestens 3 beträgt. So sind beispielsweise auch Senkungen mit quadratischem oder dreieckigem Querschnitt in der Plattenebene denkbar.

Die Senkung kann dabei als Zylinder mit einer beliebigen rotationssymmetrischen Grundfläche ausgebildet sein. Beispielsweise ist es denkbar, die Senkung als Zylinder mit quadratischer oder dreieckiger Grundfläche auszubilden. Alternativ kann die Senkung auch als Pyramide ausgebildet sein, wobei die Grundfläche ebenfalls grundsätzlich beliebig sein kann. Ebenfalls denkbar ist eine Senkung, die als Konus ausgebildet ist.

Es ist aber nicht nötig, dass die Senkung konisch oder zylindrisch ausgebildet ist. Auch kugelförmige oder ellipsoide Formen sind denkbar, insbesondere wenn die Querschnittsfläche der Senkung in der Plattenebene kreisförmig oder elliptisch ausgestaltet ist.

Alternativ ist die Senkung länglich ausgebildet. Dadurch kann das Werkzeug in der Senkung entlang einer Längsachse der Senkung geführt werden und die Platte entsprechend verschoben werden, ohne das Werkzeug aus der Senkung entfernen zu müssen.

Bevorzugt weist die Senkung eine Tiefe von 0.01 bis 2 mm , besonders bevorzugt von 0.1 bis 1 mm, noch bevorzugter von 0.2 bis 0.5 mm auf.

Bevorzugt weist die mindestens eine Senkung in mindestens einer Richtung parallel zur Oberfläche der Platte eine Grösse in einem Bereich von 0.1 bis 3 mm, besonders bevorzugt 0.5 bis 1.5 mm, noch bevorzugter 0.8 bis 1.2 mm auf.

Insbesondere kann für nicht-kreisrunde Senkungen die kürzeste Seitenwand einen Wert in den genannten Bereichen aufweisen. Insbesondere kann die mindestens eine Senkung in einer Richtung in der Ebene der Plattenoberfläche einen Wert in den genannte Bereichen aufweisen.

Bevorzugt ist die Platte in Grösse und Form an einen Knochen, insbesondere eine menschliche Clavicula, anpasst.

Bevorzugt weist die Platte daher eine Länge von 30 bis 150 mm und eine Breite von 6 bis 40 mm auf. Die Platte kann dabei in einer Ebene parallel zur Plattenoberfläche mindestens teilweise gekrümmt sein. Insbesondere kann der Krümmungsradius 30 bis 250 mm betragen.

Bevorzugt umfasst die Platte ein biokompatibles Material und besteht besonders bevorzugt daraus. Insbesondere kann das biokompatible Material aus einer Gruppe von Implantatstahl, Titan, Titanlegierungen, Keramik und Kunststoff ausgewählt sein. Besonders geeignete Kunststoffe sind insbesondere Polyetheretherketon (PEEK), UHMW-Polyethylen (PE-UHMW), Polypropylen (PP), und Polyethylenterephthalat (PET).

Es wird hierin auch beschrieben aber nicht beansprucht, ein Verfahren zur Behandlung eines Knochens, insbesondere einer Clavicula, bevorzugt einer menschlichen Clavicula. Insbesondere kann das Verfahren mit einer Platte und/oder einem Insert oder einem Kit wie vorgängig beschrieben durchgeführt werden.

Zunächst umfasst das Verfahren das Bereitstellen einer Platte. Bevorzugt umfasst die Platte mindestens eine Lasche und/oder eine Brücke, die mit der Lasche verbunden ist, wobei die Lasche und/oder die Brücke an der Platte angeordnet ist. Bevorzugt wird die Lasche und/oder die Brücke daher in einem weiteren Schritt an den Knochen angebogen.

Bevorzugt wird die Platte mittels eines Werkzeuges festgehalten, insbesondere mindestens temporär auf dem Knochen fixiert. Insbesondere kann eine Zange verwendet werden. Das Werkzeug wird bevorzugt in einer die Platte nicht durchdringenden Senkung eingebracht, wobei die Querschnittsfläche der Senkung, insbesondere in der Plattenebene, an einen Durchmesser einer Spitze des Werkzeuges angepasst ist.

Weiter wird die Platte mittels mindestens einer Schraube, die durch ein Schraubenloch in der Platte in die Clavicula eingeschraubt wird, an einer Clavicula befestigt.

Bevorzugt wird eine weitere Schraube durch mindestens eine Lasche, insbesondere durch eine Aufnahmeöffnung für eine Schraube in der Lasche, in die Clavicula eingeschraubt. Die Lasche kann insbesondere mittels einer biegbaren Brücke mit der Platte verbunden sein. Zusätzlich oder alternativ werden auf diese Weise zwei oder mehr Schrauben durch je eine Lasche oder mehrere Laschen in die Clavicula eingeschraubt. Besonders bevorzugt werden insgesamt zwei Schrauben durch eine je Lasche eingeschraubt.

Bevorzugt wird ein Insert in eine dafür angepasste Aufnahme der Platte eingesetzt. Das Insert kann dabei über eine Sollbruchstelle mit einer Halterung verbunden sein. Besonders bevorzugt wird das Insert mittels der Halterung eingesetzt und das Verfahren kann ferner einen Schritt umfassen, in dem die Halterung durch Brechen der Sollbruchstelle entfernt wird. Das Insert kann ausserdem um eine Achse, die im Wesentlichen parallel zu einer Plattenoberseite verläuft, geschwenkt werden. Insbesondere kann ein Faden durch eine Öffnung des Inserts durchgeführt werden, wobei der Faden mit einem Körperteil, bevorzugt einem Coracoid, verbunden ist. Der Faden kann insbesondere nach dem Durchführen durch die Öffnung des Inserts mit einem dafür vorgesehenen Steg verbunden werden, insbesondere durch einen Knoten.

Es kann eine weitere Schraube durch eine Öffnung des Inserts in die Clavicula eingeschraubt werden. Alternativ kann ein Faden über einen dafür vorgesehen Steg in einer Öffnung des Inserts und Fixieren des Fadens mit einem Körperteil, bevorzugt an einem Coracoid, verbunden werden. Dadurch kann der Körperteil stabilisiert werden.

Die Erfindung wird nachfolgend anhand der Figuren und Ausführungsbeispiele näher erläutert. Es zeigen:
- Fig. 1a-1c:: eine perspektivische Darstellung einer ersten Ausführungsform einer Clavicula-Platte sowie Querschnittsansichten dieser Platte senkrecht zu einer Längsrichtung.
- Fig. 2:: eine perspektivische Darstellung einer zweiten Ausführungsform einer Clavicula-Platte.
- Figs. 3a+3b:: eine perspektivische Darstellung der Clavicula-Platte von Fig. 1 mit eingesetzten Schrauben in einer Seitenansicht und einer Untersicht.
- Fig. 4:: eine perspektivische Darstellung der Clavicula-Platte von Fig. 2 mit eingesetzten Schrauben in einer Seitenansicht.
- Figs. 5a-5c:: Darstellung der Schritte eines Verfahrens zur Fixierung eines Inserts an einem Faden.
- Figs. 6a-6e:: eine erste Ausführungsform eines Inserts zur Fadenfixierung aus verschiedenen Perspektiven sowie in einem Querschnitt entlang einer Längsachse.
- Fig. 7:: die Verwendung eines Werkzeuges zum Halten der Clavicula-Platte von Fig. 2.
- Figs. 8a-8d:: die Clavicula-Platte von Fig. 7 in einer Draufsicht und in einem Querschnitt senkrecht zur Längsrichtung, sowie in 8c-8d ein nichterfindungsgemässes Verfahren zum Halten der Clavicula-Platte von Fig. 2 mit einem Werkzeug.
- Figs. 9a-9e:: eine zweite Ausführungsform eines Inserts zur Aufnahme einer Schraube in verschiedenen Perspektiven sowie in einer Querschnittsansicht entlang einer Längsachse.
- Figs. 10a-10e:: Verfahrensschritte zur Verwendung der Clavicula-Platte von Fig. 2 mit dem Insert von Figs. 9a-9e.
- Figs. 11a+11b:: Verschiedene Ausführungsformen einer Aufnahme für ein Insert, jeweils dargestellt mit und ohne Insert, in einer Draufsicht und einer Querschnittsansicht, in der das Insert jeweils leicht gekippt dargestellt ist.
- Figs. 12a-12l:: diverse alternative Inserts zur Aufnahme in einem Schraubenloch und zur Fixierung an einem Fa- den, jeweils in einer Seitenansicht, Unteransicht, Draufsicht, sowie perspektivischen Darstellung.
- Fig. 13:: die Verwendung einer Clavicula-Platte mit Inserts aus Figuren 12a, 12c und 12f.
- Figs. 14a-14h:: die Schritte eines Verfahrens zur Verwendung des Inserts von Fig. 12l.
- Fig. 15:: eine zweite Ausführungsform eines Inserts zur Fadenfixierung.
- Figs. 16a-16d:: die Schritte eines Verfahrens zur Verwendung des Inserts von Fig. 15.
- Figs. 17a+17b:: eine dritte und vierte Ausführungsform eines Inserts zur Fadenfixierung in einer Seitenansicht, Draufsicht und perspektivischen Darstellung.
- Figs. 18a-18g:: eine fünfte Ausführungsform eines Inserts zur Fadenfixierung in einer Draufsicht, perspektivischen Darstellung, Seitenansicht sowie in einer Querschnittsansicht.
- Figs. 19a-19d:: eine sechste Ausführungsform eines Inserts zur Aufnahme einer Schraube in einer Draufsicht, perspektivischen Darstellung, einer Seitenansicht sowie in einer Querschnittsansicht entlang der Längsachse.
- Fig. 20a+20b:: die Clavicula-Platte von Fig. 1 an einem Knochen fixiert in einer perspektivischen Ansicht.

Übersichtshalber sind identische Merkmale innerhalb einer Figur nicht wiederholt mit den gleichen Bezugszeichen versehen.

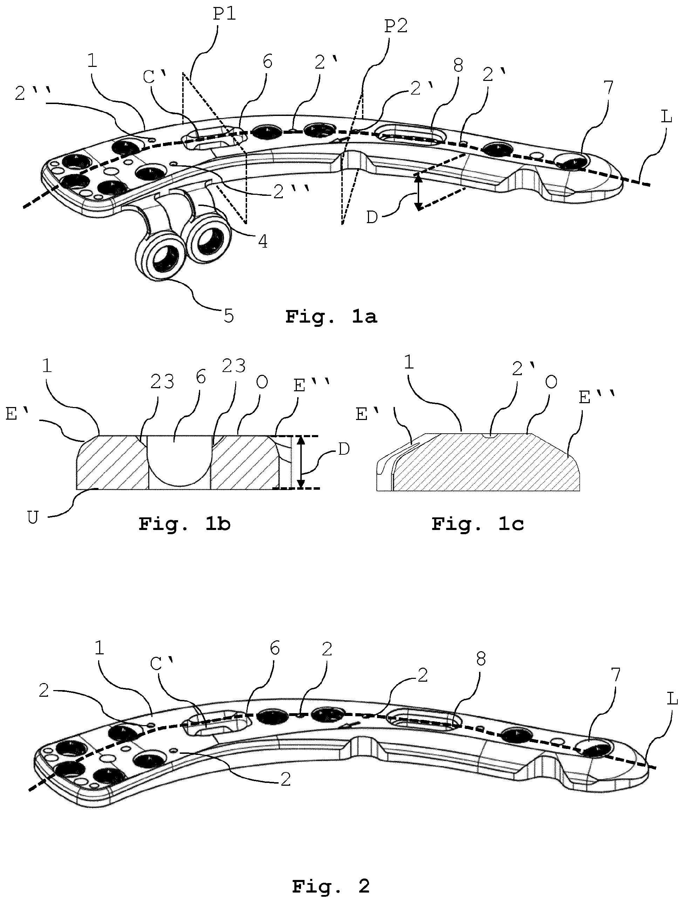

Fig. 1a zeigt eine Clavicula-Platte 1 in einer perspektivischen Darstellung. Die Platte 1 ist entlang einer Längsrichtung (L) gekrümmt und so an die Form einer menschlichen Clavicula angepasst. Die Platte weist neun runde Schraubenlöcher 7 auf. Zudem hat die Platte 1 ein Langloch 8, das im Wesentlichen entlang der Längsrichtung (L) angeordnet ist. Das Langloch 8 ist geeignet, um eine herkömmliche Knochenschraube aufzunehmen. Weiterhin umfasst die Platte 1 eine Aufnahme 6 für ein Insert (10, 20 in den folgenden Figuren). Die Aufnahme hat eine Innenkontur C', die teilweise gerundet ausgeführt ist. Vorliegend ist die Rundung als kreisbogenförmiger Querschnitt in einer Ebene P1 senkrecht zur Längsrichtung (L) ausgestaltet (siehe Fig. 1b).

Die Platte weist Senkungen 2',2'' auf ihrer Oberseite auf. In der hier dargestellten Platte sind einige Senkungen 2' entlang der Längsrichtung mittig zwischen jeweils zwei Schraubenlöchern 7,8 angeordnet. In einer Richtung senkrecht zur Längsrichtung (L) gesehen sind diese Senkungen 2' mittig zwischen den Plattenrändern angeordnet. Weitere Senkungen 2" sind in der Nähe zu Schraubenlöchern 7 angeordnet, aber nicht mittig bezogen auf die Längsrichtung (L) oder auf eine Richtung senkrecht zur Längsrichtung.

Die Senkungen haben eine kreisrunde Form. Der Durchmesser der Senkungen 2',2'' in der Ebene O (siehe Fig. 1c) beträgt 1 mm. Die Dicke D der Platte 1 variiert entlang der Längsrichtung (L). Sie ist an den Enden der Platte 1 kleiner und nimmt entlang der Längsrichtung (L) zu und erreicht im Bereich der Aufnahme 6 ein Maximum.

Die Platte 1 umfasst weiter zwei Laschen 5, die über jeweils eine Brücke 4 mit der Platte 1 verbunden sind. Die Laschen 5 sind ringförmig ausgestaltet und sind daher zur Aufnahme einer Schraube geeignet. Die Brücken 4 sind mit einem rechteckigen Querschnittsprofil ausgestaltet. Sie sind daher in einer Richtung parallel zur Längsrichtung (L) nur unwesentlich und nur mit grossen Kraftaufwand biegbar. Senkrecht zur Längsrichtung (L) sind die Brücken 4 jedoch verbiegbar. In der gezeigten Darstellung sind die Laschen 5 so angeordnet, dass eine in die Lasche 5 eingeführte Schraube im Wesentlichen senkrecht zur Längsrichtung (L) und parallel zur Plattenoberfläche angeordnet ist. Durch Biegen der Brücken 4 kann jedoch erreicht werden, dass eine eingeführte Schraube in einem Winkel ungleich 0° zur Plattenoberfläche angeordnet wäre. Durch die in Richtung der Längsrichtung (L) unwesentliche Verbiegbarkeit bleiben in die Laschen 5 eingeführte Schrauben auch bei verbogenen Brücken 4 stabil gegenüber Querkräften. Die Platte besteht aus Titan oder einer Titanlegierung.

Fig. 1b zeigt eine Querschnittsansicht der Platte 1 aus Fig. 1a entlang der Ebene P1. Die Aufnahme 6 schneidet die Ebene P1. Die Platte hat im Querschnitt eine gerade Unterseite U und eine dazu parallel verlaufende Oberseite O. Wie bereits im Zusammenhang mit Fig. 1a erläutert ist die Plattendicke D entlang der Längsrichtung L nicht konstant. Die Oberseite O und die Unterseite U sind in der Richtung der Längsrichtung L teilweise in einem Winkel zu einander angeordnet (siehe Fig. 1a, links der Ebene P1). Die Aufnahme 6 ist teilweise gerundet geformt und umfasst Aussparungen 23, deren Oberfläche konkav gewölbt ist und einen Krümmungsradius von ca. 3.5 mm aufweist. Die Aussparungen verbinden die Seitenwand der Aufnahme 6 mit der Oberseite O und sind zu beiden Seiten in einem Winkel von ca. 45° angeordnet.

Auf der Plattenoberseite sind zudem gerundete Ränder E', E" angeordnet.

Fig. 1c zeigt eine Querschnittsansicht der Platte 1 aus Fig. 1a entlang der Ebene P2. Eine Senkung 2' ist auf der Oberseite O mittig zwischen den Rändern E', E" angeordnet. Die Senkung 2' ist als Kegelstumpf ausgeführt, so dass sie einen trapezförmigen Querschnitt aufweist. Die Tiefe der Senkung beträgt 0.3 mm. Der Durchmesser der Basisfläche des Kegels in der Ebene O beträgt wie oben erwähnt 1 mm. Die versenkte Fläche der Senkung hat einen Durchmesser von 0.6 mm.

Fig. 2 zeigt eine alternative Ausführungsform einer Clavicula-Platte 1. Diese ist ähnlich der in Fig. 1a gezeigten Platte 1. Im Gegensatz zur in Fig. 1a gezeigten Platte 1 verfügt die vorliegende gezeigte Platte über keine Laschen (4 in Fig. 1a) oder Brücken (5 in Fig. 1a).

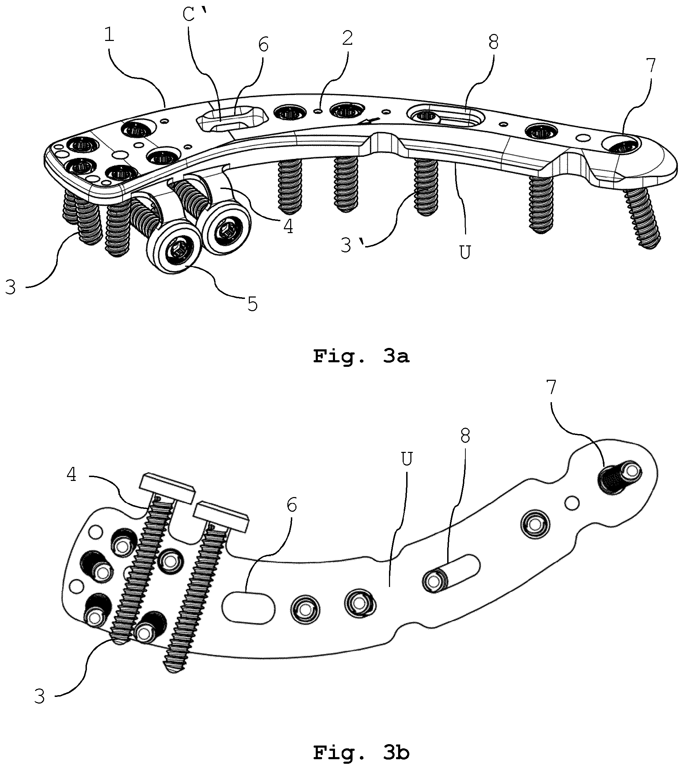

Fig. 3a zeigt die Clavicula-Platte aus Fig. 1a mit Schrauben 3, die in die Schraubenlöcher 7 eingeführt sind in einer perspektivischen Ansicht. In der vorliegend gezeigten Ausführungsform sind die Schraubenlöcher winkelvariabel ausgeführt, so dass die Schrauben jeweils in einem Winkelbereich orientiert werden können. Solche winkelvariablen Löcher sind beispielsweise in WO 2006/099766 offenbart. Alternativ wäre es aber auch möglich, die Schraubenlöcher als herkömmliche, im Wesentlichen zylinderförmig ausgestaltete Bohrungen mit einem Innengewinde auszugestalten. In diesem Fall entspräche die Orientierung der Schrauben 3 im Wesentlichen der Orientierung einer Längsachse der Löcher. Weiterhin ist eine nicht winkelstabile Schraube 3' im Langloch 8 eingeführt. Deren Schraubenkopf ist verschiebbar in Langloch 8 gelagert, so dass relativ zur Schraube 3' die Platte entlang dem Langloch 8 verschoben werden kann. In die Laschen 5 ist jeweils eine Schraube 3 eingeführt. Die Laschen 5 sind über jeweils eine Brücke 4 mit der Platte verbunden und wurden gegenüber der Ausführungsform aus 1 nicht verbogen. Daher sind die Schrauben in der gezeigten Ausführungsform parallel zur Unterseite U der Clavicula-Platte 1 orientiert.

Fig. 3b zeigt die Clavicula-Platte 1 aus Fig. 3a in einer Unteransicht. Die querenden Schrauben aus den Laschen treten zwischen den anderen Schraubachsen ohne diese zu berühren. Diese querenden Schrauben ermöglichen eine Reduktion des Risikos eines Schraubenausrisses in Knochen schlechter Qualität.

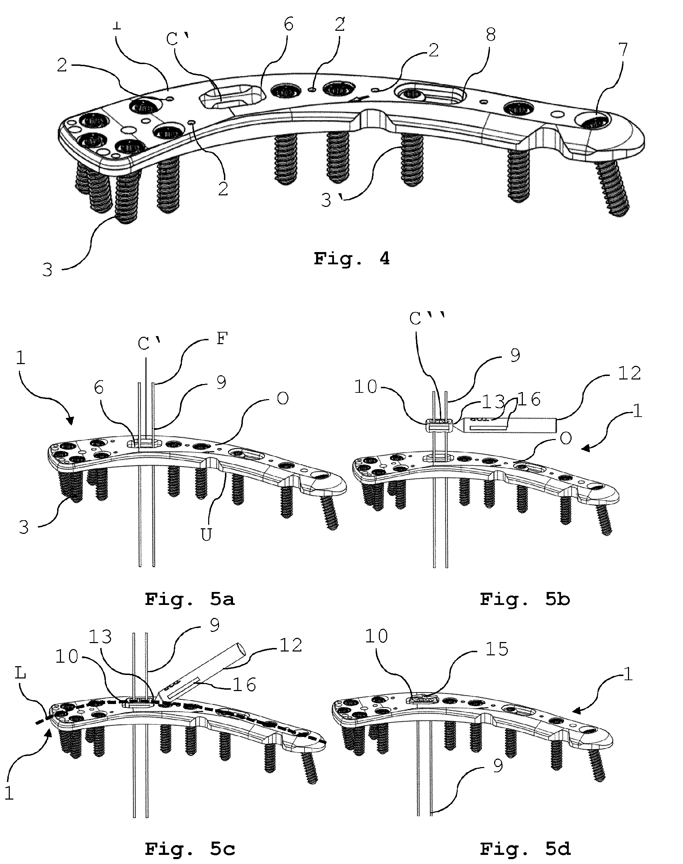

Fig. 4 zeigt die Clavicula-Platte 1 aus Fig. 2a mit in die Schraubenlöcher eingeführten Schrauben 3 in einer Schrägansicht. Die Schraubenlöcher 7 sind hier zur winkelvariablen Aufnahme von Schrauben und wie z.B. in WO 2006/099766 gezeigt ausgeführt. Die Schrauben 3 sind in einem Winkelbereich von +/-15° relativ zu einer Neutralstellung eindrehbar. Natürlich könnten alternativ auch eines oder mehrere Schraubenlöcher 7 nicht winkelvariabel ausgebildet sein. Die Schraube 3' im Langloch 8 ist verschiebbar gelagert und nicht winkelstabil, so dass sie entlang der Längsrichtung der Platte verschoben werden kann.

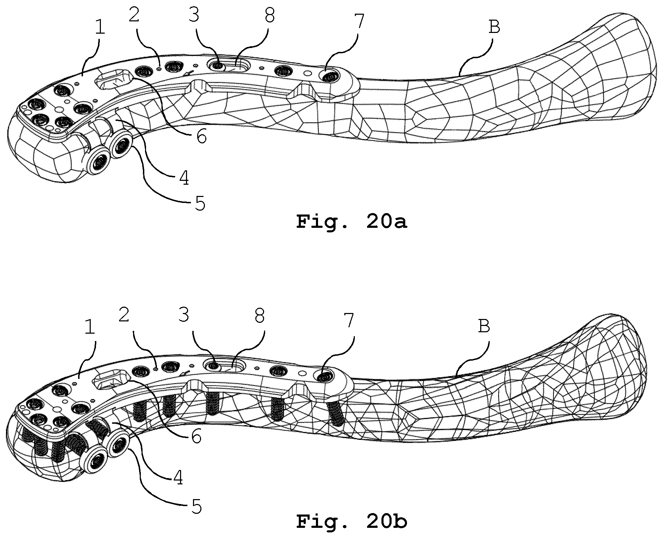

Figuren. 5a-5d zeigen ein Verfahren zur Verwendung eines Inserts 10 mit einer Clavicula-Platte 1, wie sie in Fig. 2a gezeigt ist. Selbstverständlich könnte dasselbe Verfahren auch mit anderen Ausführungsformen der Clavicula-Platte und/oder anderen als dem hier gezeigten Insert 10 und/oder an einem anderen Knochen durchgeführt werden. Weiterhin ist die Platte 1 übersichtshalber ohne Knochen (siehe Figuren 20a+20b) dargestellt. Das Verfahren könnte aber natürlich durchgeführt werden, nachdem die Schrauben 3 in einen Knochen eingeschraubt wurden.

Fig. 5a zeigt, wie zunächst ein Faden 9 durch die Aufnahme 6 geführt wird. Der Faden 9 kann vorliegend aus UHMWPE bestehen. Der Faden 9 hat zwei freie Enden F, die von der Plattenunterseite U her durch die Aufnahme 6 geführt werden.

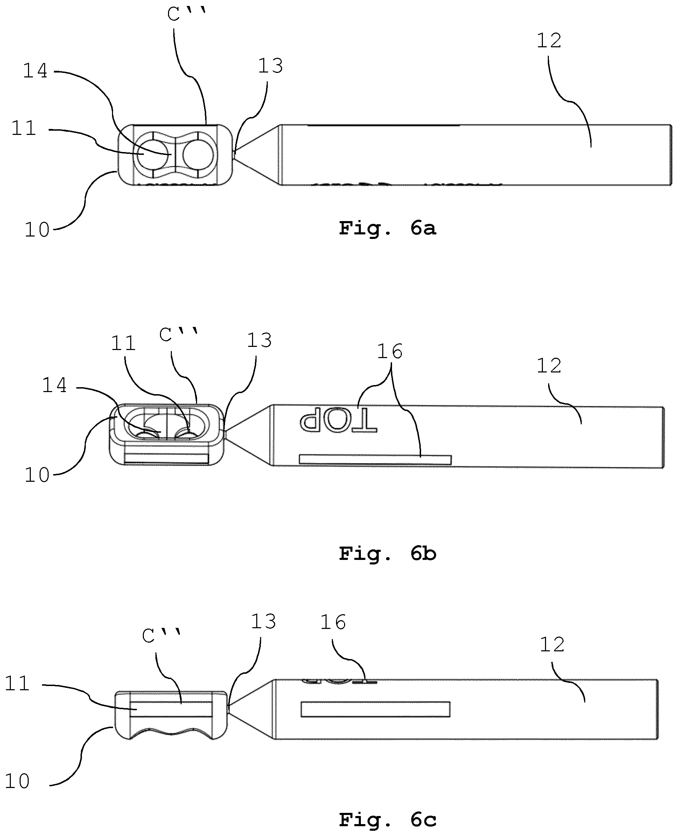

Fig. 5b zeigt, wie der Faden 9 anschliessend durch ein Insert 10 geführt wird. Das Insert 10 ist zur Fixierung an einem Faden 9 angepasst und verfügt dafür über einen versenkten Steg (nicht sichtbar) und eine gerundete Aussenkontur C". Das Insert 10 ist über eine Sollbruchstelle 13 mit einer Halterung 12 verbunden (siehe Figuren 6a-6c). Die Halterung weist zwei Markierungen 16 auf, die einerseits dem Benutzer anzeigt, welche Seite des Inserts 10 im eingesetzten Zustand in die gleiche Richtung wie die Plattenoberseite O zeigen soll und andererseits eine Seriennummer aufweist. Letztere kann während der Behandlung aufbewahrt werden und ermöglicht so eine einfachere Dokumentation der Behandlung. Dies verringert die Fehlerrate und erhöht damit die Sicherheit der Behandlung. Der Faden 9 wird mit je einem Ende F auf beiden Seiten des Steges (siehe beispielsweise Figuren 6a+6b) durch das Insert 10 geführt.

Fig. 5c zeigt, wie das Insert 10 mit Hilfe der Halterung 12 in die Aufnahme 6 der Clavicula-Platte geführt wird. Die Halterung 12 kann vom Insert 10 entfernt werden, indem die Sollbruchstelle 13 durch Biegen gebrochen wird. Die Halterung 12 kann nach dem Entfernen aufbewahrt werden und erlaubt es, über die Markierung 16, die eine Seriennummer umfasst, das eingesetzte Insert auch zu einem späteren Zeitpunkt zu identifizieren. Vorliegend sind die Innenkontur C' der Aufnahme 6 teilweise und die Aussenkontur C" des Inserts 10 ganz im Querschnitt kreiszylindrisch ausgebildet, wodurch das Insert 10 in der Aufnahme 6 um die Achse des Inserts 1 und/oder der Aufnahme 6 verschwenkt werden kann.

Fig. 5d zeigt, wie der Faden 9 schliesslich durch einen Knoten 15 am Insert 10 befestigt wird. Es ist auch denkbar, mehrere Knoten zu verwenden. Wird der Faden 9, beispielsweise über einen weiteren Knoten oder über eine Schlaufe an einem Coracoid befestigt, ist dieses über das Insert 10 der Clavicula-Platte 1 verbunden, so dass der Abstand zwischen dem Coracoid und der Clavicula konstant und physiologisch korrekt gehalten wird.

Fig. 6a zeigt ein Insert 10, wie es im Verfahren gemäss Figuren 5a-5d verwendet werden kann, in einer Unteransicht. Das Insert 10 ist zur Fixierung mit einem Faden angepasst und verfügt daher über einen Steg 14. Dieser ist versenkt ausgeführt und bildet zwei Öffnungen 11, durch die ein Faden 9 geführt werden kann, so dass dieser mittels eines Knotens oder einer Schlaufe mit dem Steg 14 verbunden werden kann. Das Insert 10 ist über eine Sollbruchstelle 13 mit einer Halterung 12 verbunden. Das Insert 10 besteht vorliegend aus einer Titanlegierung und ist einstückig mit der Halterung 12 verbunden.

Fig. 6b zeigt das Insert von Fig. 6a in einer perspektivischen Ansicht. Die Halterung 12 weist zwei Markierungen auf. Einerseits zeigt eine Makierung mit "TOP" an, dass diese Seite beim Einsetzen in eine Aufnahme 6 einer Clavicula-Platte 1 in die gleiche Richtung wie die Oberseite der Platte 1 zeigen soll. Anderseits ist eine zweite Markierung 16 beispielsweise in der Form einer Seriennummer oder eines Barcodes angebracht, der zur Identifizierung des Inserts 10 vor, während, und nach dessen Einsatz dient.

Fig. 6c zeigt das Insert 10 von Figuren 6a und 6b in einer Seitenansicht.

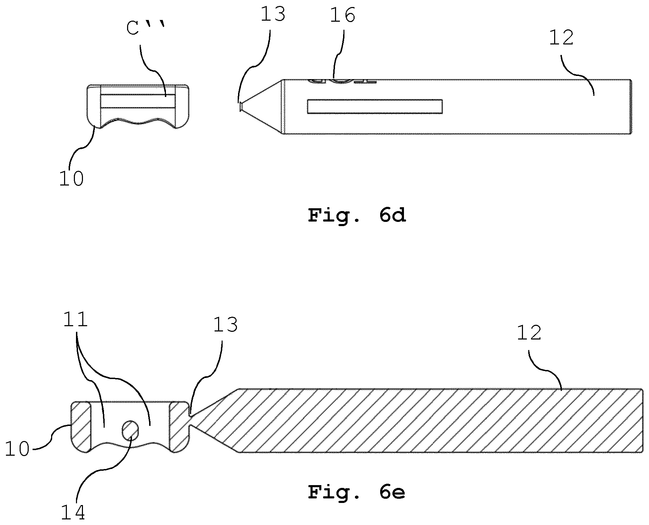

Fig. 6d zeigt das Insert 10 von Figuren 6a-6c in der gleichen Seitenansicht wie in Fig. 6c. Die Sollbruchstelle 13 ist gebrochen, so dass das Insert 10 und die Halterung nicht mehr miteinander verbunden sind.

Fig. 6e zeigt das Insert von Fig. 6c in einer Querschnittsansicht entlang einer Längsachse des Inserts 10 und der Halterung 12. Der Steg 14 ist versenkt angeordnet und bildet zwei Öffnungen 11, durch die ein Faden 9 geführt werden kann.

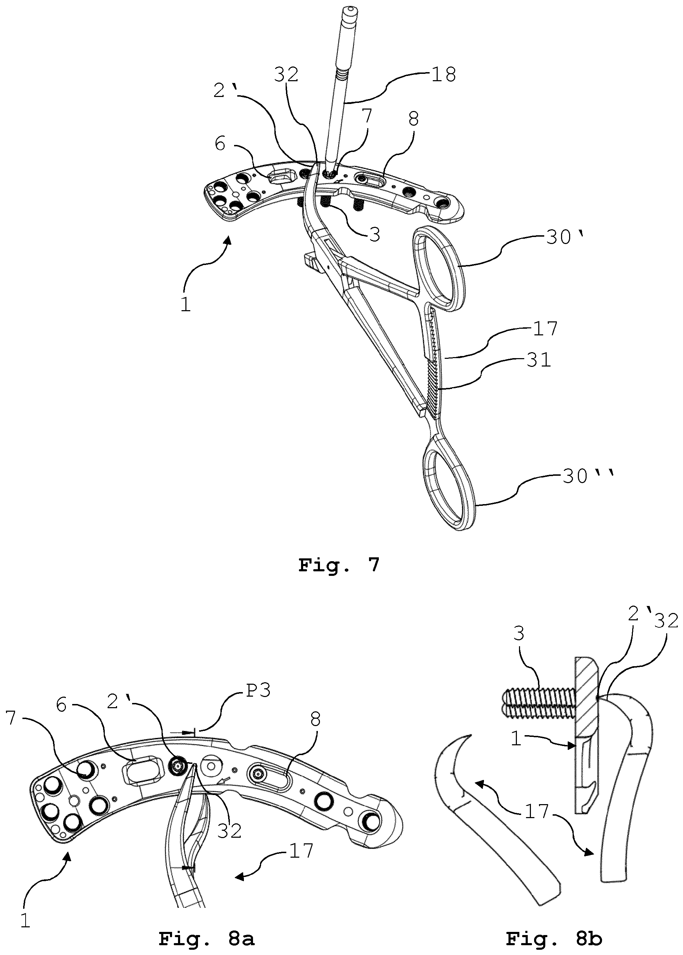

Fig. 7 zeigt schematisch das Halten einer Clavicula-Platte 1 mit Hilfe eines Werkzeuges 17. Das Werkzeug 17 ist vorliegend eine chirurgische Zange, die einen Handgriff 30',30" umfasst, der mit zwei Fingern gegriffen werden kann. Die Zange 17 umfasst weiter einen Arretierungsraster 31 und mindestens eine Spitze 32. Die Spitze 32 kann in eine Senkung 2' eingebracht werden und ist daher mittels eines Formschlusses daran gehindert, sich relativ zur Platte 1 zu bewegen. Durch die Arretierungsraster 31 kann die Öffnung der Zange ausserdem in dieser Position arretiert werden, so dass die Platte temporär fixiert ist und ein Benutzer, z.B. ein Arzt, die Zange für die weitere Behandlung nicht halten muss. So hat er die Hände frei, um beispielsweise eine Knochenschraube 3 mittels eines Schraubendrehers 18 durch ein Schraubenloch 7 in den Knochen zu schrauben.

Fig. 8a zeigt eine Draufsicht der Spitze 32 eines Werkzeuges 17, das in die Senkung 2' eingebracht 6 ist, wie sie in Fig. 7 gezeigt ist. Da die Zange in der Senkung 2' zwischen Plattenlöchern gesetzt ist, kann ohne Probleme eine Knochenschraube in diese Plattenlöcher geschraubt werden.

Fig. 8b zeigt eine Querschnittsansicht des Werkzeuges 17 und der Platte 1 entlang der Ebene P3 aus Fig. 8a. Das Werkzeug umfasst zwei Spitzen 32, die im Wesentlichen identisch ausgeführt sind. So lässt sich das Werkzeug 17 mit beiden Spitzen 32 in eine Senkung 2' einführen. Alternativ wäre es auch möglich, eine Seite des Werkzeuges mit einem anderen Ende auszuführen. Beispielsweise könnte eine flacher Greifer benutzt werden, um Gewebe greifen zu können und gleichzeitig die Verletzung dieses Gewebes zu reduzieren.

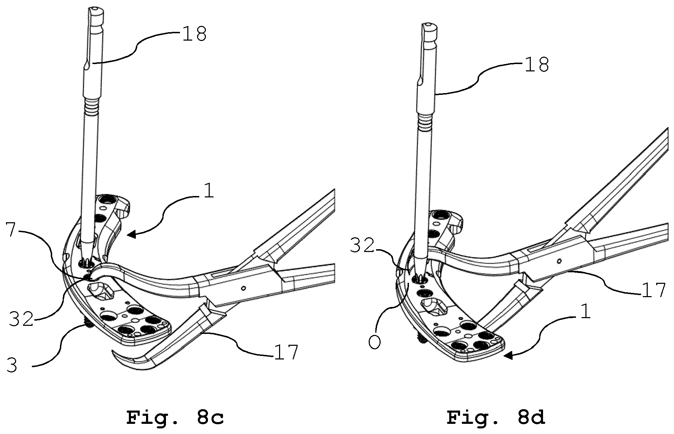

Fig. 8c zeigt ein nicht erfindungsgemässes Verfahren zum Halten einer Platte 1 mit einem Werkzeug. Hierbei wird eine Spitze 32 des Werkzeugs in ein Schraubenloch 7 eingeführt, um die Platte 1 temporär zu fixieren. Zwar wird dadurch die Bewegung der Platte 1 relativ zum Werkzeug 17 eingeschränkt. Allerdings ist eine gewisse Bewegung immer noch möglich, da das Schraubenloch 7 eine nicht an das Werkzeug 17 angepasste Grösse und Form aufweist. Ausserdem kann in das benutzte Schraubenloch 7 zumindest temporär keine Schraube 3 eingesetzt werden.

Fig. 8d zeigt ein weiteres nicht erfindungsgemässes Verfahren zum Halten einer Platte 1 mit einem Werkzeug. Hierbei wird die Spitze 32 auf der Plattenoberseite O aufgesetzt. Die Platte 1 und das Werkzeug 17 sind dabei lediglich über einen Kraftschluss verbunden. Die Platte 1 kann daher leicht verrutschen. Zusätzlich können sich die Spitze 32 und die Platte beschädigen, z.B. verkratzen.

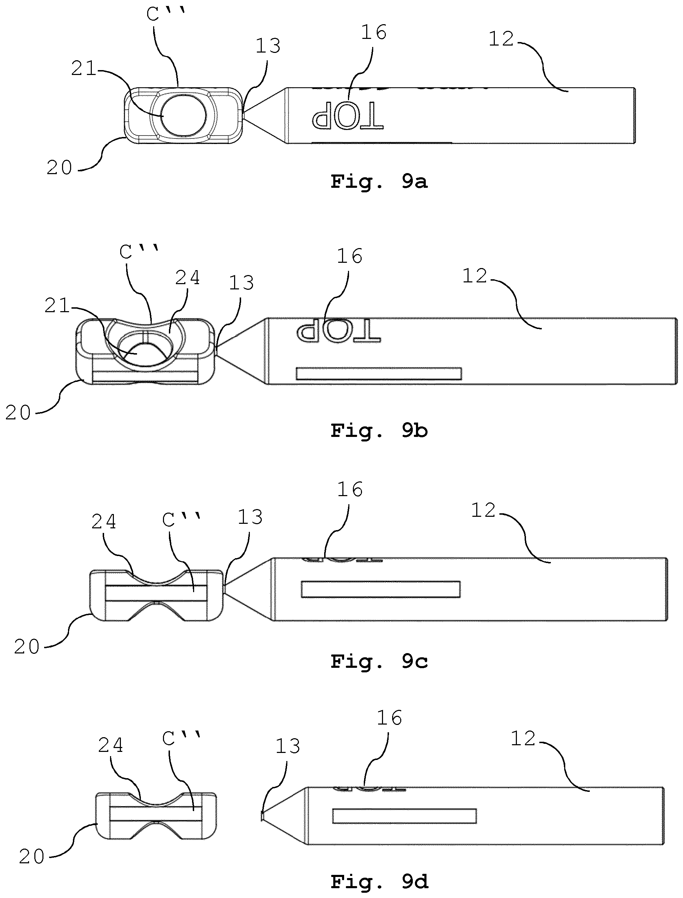

Fig. 9a zeigt eine zweite Ausführungsform eines Inserts 20 in einer Draufsicht. Dieses ist angepasst, um eine Schraube aufzunehmen. Dazu verfügt es über eine Öffnung 21, in die eine Schraube eingeführt werden kann. Das Insert 20 ist über eine Sollbruchstelle 13 mit einer Halterung 12 verbunden, die zwei Markierungen 16 aufweist. Die Halterung 12 entspricht im Wesentlichen der Halterung von Fig. 6a.

Fig. 9b zeigt das Insert 20 von Fig. 9b in einer perspektivischen Ansicht. Die Öffnung 21 zur Aufnahme einer Schraube ist hier ohne Gewinde ausgeführt. Es wäre aber natürlich denkbar, dass die Öffnung ein Innengewinde oder eine andere Kontur zur winkelstabilen Aufnahme einer Schraube, insbesondere eine Aufnahme gemäss WO 2006/099766 aufweist. Die Öffnung hat eine im Wesentlichen zylindrische Form und ist ausserdem als Senklochbohrung 24 ausgeführt.

Fig. 9c zeigt das Insert von Figuren 9a und 9b in einer Seitenansicht. Die Senklochbohrung 24 ist so ausgebildet, dass das Insert 20 in seitlicher Ansicht Vertiefungen aufweist.

Fig. 9d zeigt das Insert 20 von Figuren 9a-9c, wobei die Sollbruchstelle 13 gebrochen wurde und die Halterung 12 daher vom Insert 20 getrennt ist.

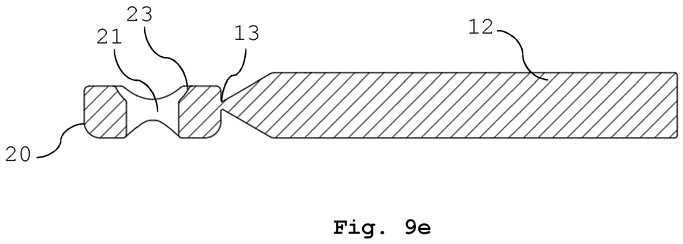

Fig. 9e zeigt das Insert von Figuren 9a-9d in einer Querschnittsansicht entlang der Längsachse der Halterung 12 und des Inserts 20.

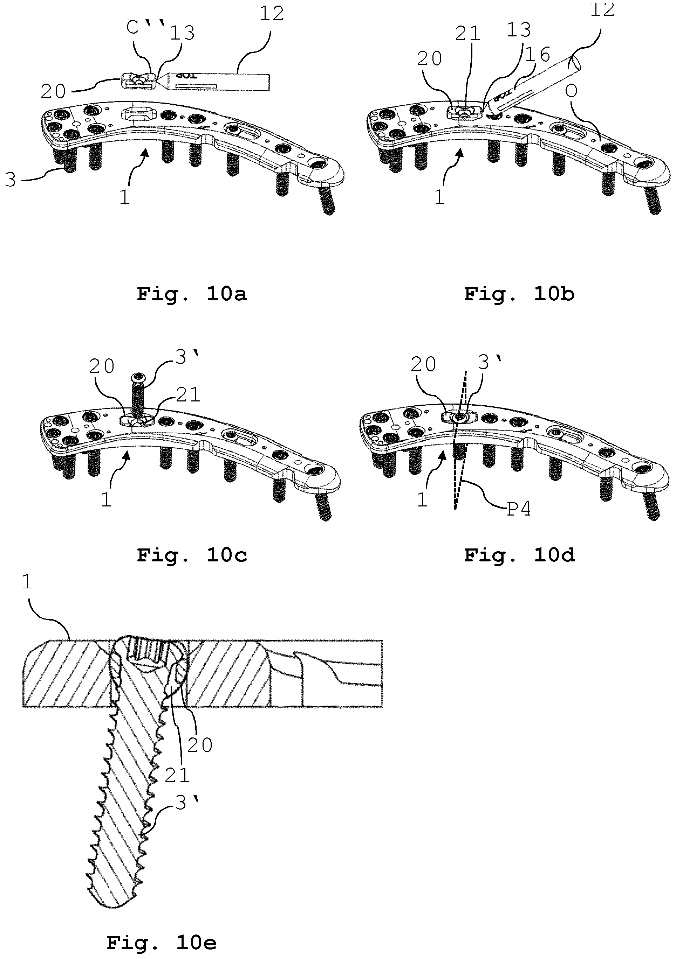

Die Figuren 10a-10e zeigen schematisch ein Verfahren zur Verwendung des Inserts 20 aus Figuren 9a-9e zur Verwendung mit einer Schraube 3' mit einer Clavicula-Platte 1, wie sie in Fig. 2a gezeigt ist. Selbstverständlich könnte dasselbe Verfahren auch mit anderen Ausführungsformen der Clavicula-Platte 1 und/oder anderen als dem hier gezeigten Insert 20 und/oder an anderen als dem hier gezeigten Knochen durchgeführt werden. Weiterhin ist die Platte 1 übersichtshalber ohne Knochen (siehe Figuren 20a+20b) dargestellt. Das Verfahren wird typischerweise durchgeführt, nachdem die Schrauben 3 in einen Knochen eingeschraubt wurden.

Fig. 10a zeigt die Platte 1 mit in die Schraubenlöcher 7,8 eingeschraubten Schrauben 3. Das Insert 20 entspricht im Wesentlichen dem von Figuren 9a-9e.

Fig. 10b zeigt, wie das Insert 20 in eine dafür vorgesehene Aufnahme 6 eingeführt wird. Dabei zeigt die Markierung 16 an, wie das Insert orientiert werden muss, d.h. mit der Markerung "TOP" 16 in die gleiche Richtung wie die Oberseite O der Platte 1 zeigend. Es ist aber möglich, das Insert 20 in der Aufnahme 6 zu schwenken. In diesem Fall kann die Öffnung 21 so orientiert werden, dass eine in sie eingesetzte Schraube 3 in einem gewünschten Winkel in einen Knochen geschraubt werden kann. Durch Wegbiegen der Halterung 12 kann die Sollbruchstelle 13 gebrochen werden und das Insert 20 wird so von der Halterung 12 getrennt.

Fig. 10c zeigt, wie eine Schraube 3' in die Öffnung 21 des Inserts 20 eingeführt wird.

Fig. 10d zeigt, wie die Schraube 3' vollständig in die Öffnung 21 des Inserts 20 eingeführt wurde.

Fig. 10e zeigt einen Querschnitt des Inserts 20, der Schraube 3' und der Platte 1 von Fig. 10d in der Ebene P4. Die Schraube 3' ist schräg zur Platte 1 orientiert, da das Insert 20 in der Aufnahme 6 geschwenkt wurde. Die Öffnung 21 des Inserts 20 ist so ausgeführt, dass die Schraube 3' in der Öffnung 21 Spiel hat und bei Bedarf auch relativ zur Öffnung 21 und zum Insert 20 geschwenkt werden könnte. Dies wird insbesondere auch dadurch erreicht, dass die Öffnung einen grösseren Durchmesser aufweist als der Schaft der Schraube 3'.

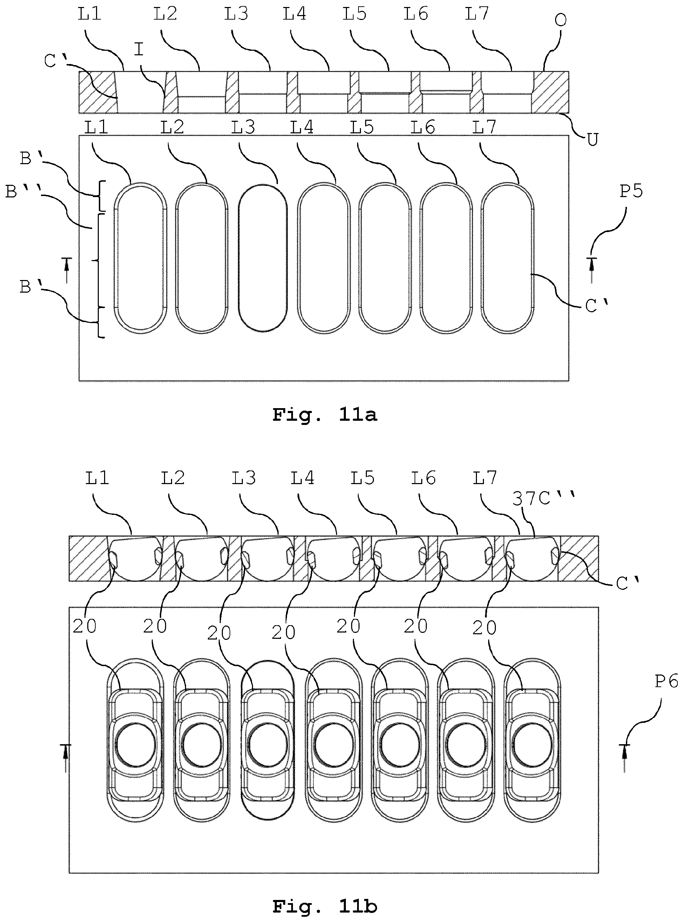

Fig. 11a zeigt diverse Aufnahmen L1,L2,L3,L4,L5,L6,L7,L8. Diese sind zur besseren Illustration und übersichtshalber nicht in einer Clavicula-Platte 1 gezeigt. Jede der gezeigten Aufnahmen L1,L2,L3,L4,L5,L6,L7,L8 könnte aber mit jeder der hier beschriebenen Clavicula-Platten 1, insbesondere den von Figuren 1a und 2a, kombiniert werden und ist zur Aufnahme eines Inserts 10,20 geeignet. Die Aufnahmen L1,L2,L3,L4,L5,L6,L7,L8 sind unten in einer Draufsicht gezeigt. Direkt oberhalb ist jeweils ein Querschnitt der jeweiligen Aufnahme L1,L2,L3,L4,L5,L6,L7,L8 in der Ebene P5 gezeigt. Alle Aufnahmen L1,L2,L3,L4,L5,L6,L7,L8 sind an zwei Endabschnitten B' jeweils gerundet geformt. Ein Mittelbereich B" weist hingegen jeweils einen in der Draufsicht geraden Abschnitt, insbesondere mit zwei spiegelsymmetrischen Seitenwänden auf.

Allen Aufnahmen L1,L2,L3,L4,L5,L6,L7,L8 gemeinsam ist, dass sich eine Innenwand I von einer Oberseite zu einer Unterseite hin zumindest teilweise verjüngt. Ausserdem ist der Endabschnitt B' gerundet und der Mittelbereich B" in der Draufsicht gerade mit spiegelsymmetrischen Seitenflächen ausgebildet.

Die Aufnahme L1 weist im Mittelbereich B" einen trapezförmigen Querschnitt auf. Die Innenwände I im Mittelbereich B' sind als gerade Flächen ausgebildet, die in einem Winkel zueinander stehen, so dass sie sich von der Oberseite O zur Unterseite U hin verjüngen. Die Aufnahme L1 ist also im Mittelbereich B" prismenförmig ausgebildet.

Aufnahme L2 ist im an die Unterseite U angrenzenden Bereich nicht verjüngend ausgebildet. Stattdessen sind die Innenwände I im Mittelbereich B" teilweise (im an die Unterseite U angrenzenden Bereich) parallel zueinander ausgebildet. Direkt darüber, im an die Oberseite O angrenzenden Bereich, ist die Aufnahme im Wesentlichen gleich geformt wie die Aufnahme L1.

Aufnahme L3 hat eine teilweise sphärische und eine teilweise zylindrische Innenfläche I. Der Krümmungsradius der sphärischen Innenfläche I entspricht dem Krümmungsradius der Aussenkontur C" des Inserts 1 (siehe Fig. 11b).

Aufnahme L4 weist einen Querschnitt auf, der zwei Rechtecken entspricht. Hierbei ist das an der Oberseite O angeordnete Rechteck breiter ausgestaltet. Die beiden Bereiche mit unterschiedlicher Breite sind durch eine Grenzfläche parallel zur Oberseite O getrennt.

Aufnahme L5 ist ähnlich der Aufnahme L4, wobei die beiden Bereiche mit unterschiedlicher Breite aber durch eine Grenzfläche getrennt sind, die nicht parallel zur Oberseite O ausgestaltet ist.

Aufnahme L6 ist ähnlich der Aufnahme L3. Vorliegend ist aber der Krümmungsradius der sphärischen Innenfläche I unterschiedlich zum Krümmungsradius der Aussenkontur C" des Inserts.

Aufnahme L7 ist ähnlich den Aufnahmen L4 und L5, wobei die zwei Bereiche mit unterschiedlicher Breite durch einen elliptischen Abschnitt getrennt sind.

Die hier gezeigten Formen der Querschnitte sind nur beispielhafter Natur und könnten auch andere Formen aufweisen. Denkbar sind insbesondere hyperbolische, parabolische, und/oder mehrfach gestufte Ausführungen.

Fig. 11b zeigt die Aufnahmen L1,L2,L3,L4,L5,L6,L7,L8 von Fig. 11a mit einem eingesetzten Insert 20 zur Aufnahme einer Schraube. Das beispielhaft gezeigte Insert 20 entspricht dem Insert 20 von Fig. 9a, es ist aber jedes der hier beschriebenen Inserts 10,20 zur Aufnahme in jede der gezeigten Aufnahmen 6, L1, L2, L3, L4, L5, L6, L7, L8 geeignet. Die Aussenkontur C" der Inserts 20 ist gerundet ausgebildet. Der Querschnitt des Inserts 20 senkrecht zu seiner Längsachse ist ein Kreissegment, da das Insert 20 einen abgefrästen Abschnitt 37 umfasst, der im Wesentlichen gerade ist. Die gerundete Aussenkontur C' des Inserts 20 ist daher so auf alle gezeigten Innenkonturen C" angepasst, dass das Insert 20 in einer Ebene senkrecht zur Längsachse des Inserts 20 und der Aufnahme L1,L2,L3,L4,L5,L6,L7,L8 geschwenkt werden kann. Zudem ist das Insert entlang der Längsachse der Aufnahme 6 verschiebbar und kann nicht durch die Aufnahme rutschen.

Alternativ könnte in die in den Figuren 11a und 11b gezeigten Aufnahmen L3, L6, L7 auch ein Insert 10,20 mit einer anderen Form, beispielsweise einer eckigen Form eingesetzt werden. Die Schwenkbarkeit wäre dank der teilweise gerundeten Innenkontur C' der Aufnahme L3, L6, L7 gewährleistet.

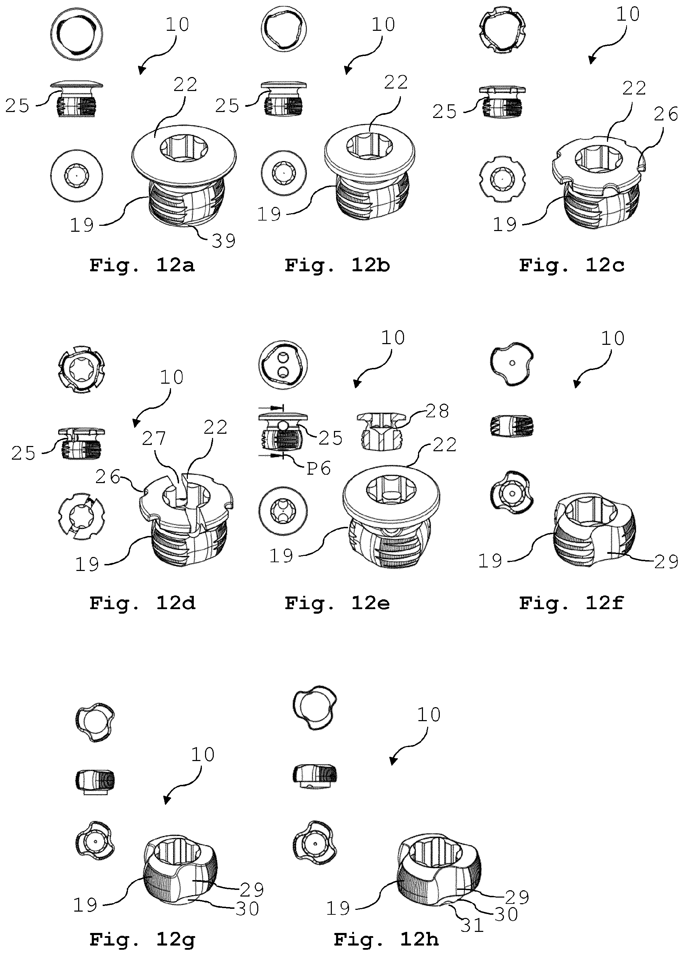

Figuren 12a-12l zeigen diverse Inserts 10 zur Fadenfixierung, die zur Aufnahme in ein Schraubenloch 7 gemäss WO 2006/099766 geeignet sind. Zur Verwendung der in den Figuren 12a-12l gezeigten Inserts ist also keine speziell dafür angepasste Aufnahme 6 notwendig.

Die in den Figuren 12a-12l gezeigten Inserts 10 sind angepasst, um in ein herkömmliches Schraubenloch einer Platte eingeschraubt zu werden. Sie weisen dafür einen Verblockungsbereich gemäss WO 2006/099766 und eine Aufnahme für ein Werkzeug auf. Bevorzugt ist die Aufnahme als Torx-Antrieb ausgeführt. Das Insert 10 ist zum Halten eines Fadens angepasst und weist hierzu bevorzugt mindestens eine Bohrung und/oder einen Kopf mit einen Hinterschnitt auf. Bevorzugt ist der Verblockungsbereich so geformt, dass im eingedrehten Zustand mindestens ein Bereich mit einem Abstand zur Innenform eines Schraubenloches entsteht, durch den ein Faden geführt werden kann. Bevorzugt weist der Kopf mindestens eine Aussparung auf, durch den ein Faden geführt werden kann. Ein derartiges Insert ermöglicht es insbesondere, einen Faden mittels eines Knotens zu fixieren, so dass der Knoten in einem Schraubenloch versenkt angeordnet ist.

Fig. 12a zeigt eine Ausführungsform eines Inserts 10 mit einem Verblockungsbereich 19 zur Aufnahme in ein winkelvariables Schraubenloch 7 wie in WO 2006/099766 gezeigt. Das Insert 10 kann daher in verschiedenen Winkeln in ein Schraubenloch eingeführt werden. Es umfasst einen Kopfbereich 22 sowie einen darunter anliegenden Halsbereich 25. Dieser umfasst einen Zentrierbund, an dem ein Faden befestigt werden kann und der über einen kreiszylindrischen Abschnitt mit dem Verblockungsbereich 19 verbunden ist. Der Faden wird bei dieser Ausführung über die Plattenkante geführt. Das hier gezeigte Insert weist an der Unterseite einen Zentrierbund 39 auf. Dieser ermöglicht, dass sich das Insert in einem Loch nach der Lochachse ausrichten kann und weniger Gefahr läuft, bei Zug am Faden abgekippt zu werden.

Fig. 12b zeigt ein Insert 10, das ähnlich dem in Fig. 12a gezeigten Insert 10 ist. Der Halsbereich ist vorliegend sphärisch ausgestaltet. Der Faden wird bei dieser Ausführung über die Plattenkante geführt. Das hier gezeigte Insert weist im Gegensatz zur Ausführungsform von Fig. 12a keinen Zentrierbund auf.

Fig. 12c zeigt eine weitere Ausführungsform eines Inserts 10 zur Fadenfixierung. Es umfasst einen Kopfbereich 22 sowie einen gebogenen Halsabschnitt 25, der direkt an den Verblockungsbereich 19 angrenzt. Dem gezeigten Insert 10 fehlt also ein kreiszylindrischer Abschnitt (siehe Figuren 12a und 12b), wodurch ein am Halsbereich 25 gehaltener Faden bei Bedarf in ein Schraubenloch 7 versenkt werden kann. Der Kopfbereich 22 umfasst sechs Einkerbungen 26, durch die der Faden insbesondere bei Versenkung des Inserts durchgeführt werden kann. Der Faden kann bei dieser Ausführung über die Plattenkante geführt werden.

Fig. 12d zeigt ein Insert 10, das ähnlich dem in Fig. 12c gezeigten ist. Der Kopfbereich 22 umfasst vier Einkerbungen und einen Schlitz 27. Das Insert ist durchbohrt, d.h. der Faden kann durch den Knochen geführt werden. Der Schlitz 27 ermöglicht es, den Faden beim Eindrehen des Inserts in das Plattenloch seitlich neben der Schraubendreherklinge vorbeizuführen und ihn nachträglich am Hals des Inserts zu befestigen.

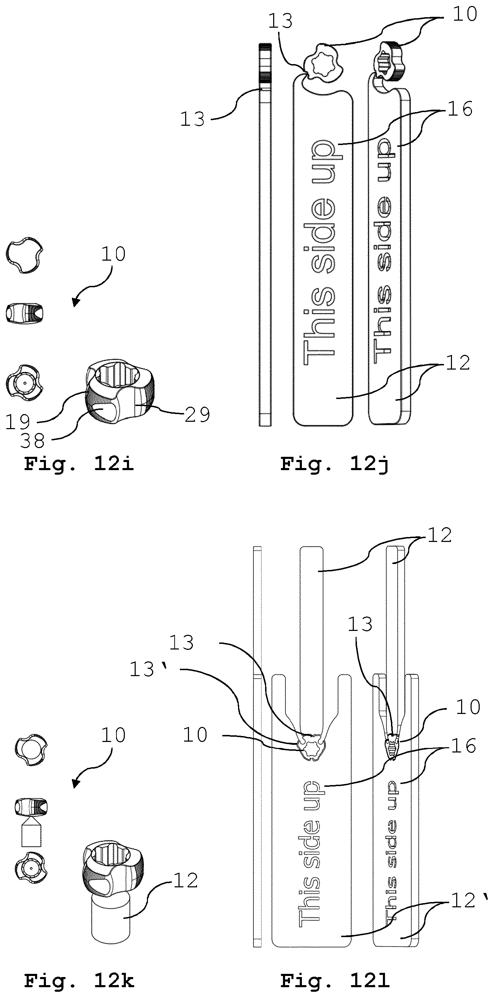

Fig. 12e zeigt ein Insert 10, das ähnlich dem im Fig. 12 gezeigten Insert 10 ist und weiterhin zwei Bohrungen 28 umfasst. Die zwei Bohrungen sind bezüglich der Ebene P6 symmetrisch ausgestaltet und verlaufen im Verblockungsbereich 19 parallel zu der Ebene P6. Im Halsbereich 25 verlaufen die Bohrungen 28 senkrecht zu den Bohrungen im Verblockungsbereich 19. Dadurch ist dieses Insert 10 geeignet, um einen Faden durch die eine Bohrung 28 aufzunehmen, so dass er um den Halsabschnitt 25 gewickelt werden kann und durch die andere Bohrung 28 wieder ausgeführt werden kann. Ebenfalls ermöglicht diese Anordnung das Einstecken einer Schraubendreherklinge.

Fig. 12f zeigt ein Insert 10, das der verblockbaren winkelvariablen Anordnung in einem Schraubenloch 7 dient (wie in WO 2006/099766 gezeigt). Eine Fadenfixierung kann vorgenommen werden, indem ein Faden zwischen zwei Verblockungsabschnitten 29 seitlich am Insert 10 vorbeigeführt wird. Durch die Verankerung des Inserts 10 in einer Platte (nicht gezeigt) mittels des Verblockungsbereichs 19 ist der Faden seitlich gehalten und kann über dem Insert beispielsweise durch einen Knoten gehalten werden.

Fig. 12g zeigt ein Insert 10 ähnlich dem in Fig. 12f gezeigten. Dieses Insert 10 umfasst zusätzlich einen Bund 30 zum besseren Abgreifen des Inserts während der Produktion auf einer Maschine.

Fig. 12h zeigt ein Insert 10 ähnlich dem in Fig. 12g gezeigten. Dieses umfasst zusätzlich am Bund 20 eine Aussparung 31. Diese ermöglicht ist, nach dem Einschrauben in ein Schraubenloch 7 einen Faden aufzunehmen und so verletzungsfrei in den Abschnitt 29 zwischen den Verblockungsbereichen zu führen.

Fig. 12i zeigt ein Insert 10 ähnlich dem in Fig. 12f gezeigten. Es umfasst zusätzlich im Verblockungsbereich 19 eine Einfräsung 38 zum besseren Abgreifen während der Produktion auf einer Maschine.

Fig. 12j zeigt eine weitere Ausführungsform eines Inserts 10, das zur Fadenfixierung in ein herkömmliches Schraubenloch 7 eingeführt werden kann. Dieses ist über eine Sollbruchstelle 13 mit einer Halterung 12 verbunden. Dieses ist durch laserschneiden als Ausschnitt aus einem Blech gebildet und umfasst weiterhin eine Markierung 16, die zur Orientierung des Inserts 10 in einem Schraubenloch 7 dient.

Fig. 12k zeigt ein Insert 10, das im Wesentlichen wie das in Fig. 12i gezeigte Insert 10 ausgestaltet ist. Es umfasst zusätzlich eine Halterung 12, die zur Trennung vom Insert 10 abknickbar ist.

Fig. 12l zeigt ein Insert 10 ähnlich dem in Fig. 12j gezeigten. Die Halterung 12,12' umfasst zwei Teile, die über jeweils eine Sollbruchstelle 13,13' mit dem Insert 10 verbunden sind. Die zweistückige Halterung 12,12' ist durch Laserschneiden aus einem Blech gebildet.

Das Insert in Fig. 12l weist zwei Halterungen auf. Zusammen formen die Halterungen eine Führung für einen Faden, der bevorzugt am Insert gehalten werden kann. Beide Halterungen sind über jeweils eine Sollbruchstelle mit dem Insert verbunden, so dass sie unabhängig voneinander gebrochen werden können. Bevorzugt weist das Insert eine Ausnehmung auf, in der ein Faden aufgenommen werden kann. Besonders bevorzugt ist der Faden in der Ausnehmung nicht ohne Kraftaufwand entfernbar.

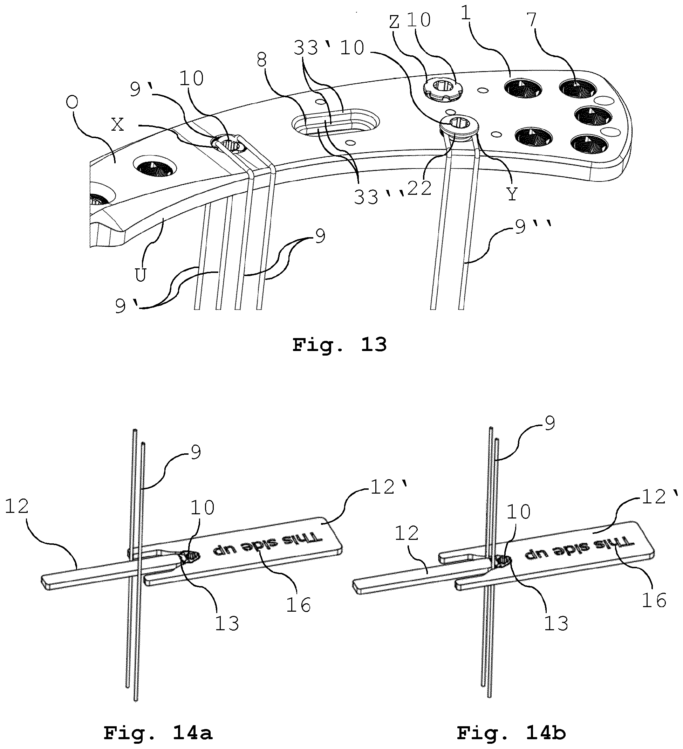

Fig. 13 zeigt schematisch die Verwendung von Inserts 10 aus Fig. 12 zur Fadenfixierung in einem herkömmlichen Schraubenloch 7 in einer Clavicula-Platte.

Die Clavicula-Platte 1 umfasst neben mehreren Schraubenlöchern 7 auch Langloch 8.

An einer Position X ist das Insert 10 von Fig. 12f eingeführt. Alternativ könnte auch ein Insert der Figuren 12g-12l analog verwendet werden. Dieses verankert zwei Fäden 9,9', die von je einer Seite als Fadenschlinge in das Plattenloch gesteckt wurden. Nach Eindrehen des Inserts sind sie vor dem Verlieren geschützt und der Faden 9wird über die Plattenkante geführt und kann dort beispielsweise verknotet werden. Der Faden 9' ist analog, aber in umgekehrter Richtung im Plattenloch fixiert und ist daher nicht über die Plattenkante geführt. Alternativ könnten die Fäden 9,9' durch den Knochen geführt, mittels Insert im Plattenloch vor dem Verlieren geschützt und über dem Insert geknotet werden.

An einer Position Y ist das Insert 10 von Fig. 12a eingeführt. Der Faden 9" umfasst eine Schlaufe, die im Halsbereich 25 des Inserts 10 verankert ist. Das Insert 10 ist zur besseren Übersicht in einem nicht vollständig eingeschraubten Zustand dargestellt. Durch weiteres Einschrauben kann der Faden 9" gegen die Plattenoberseite O gepresst werden. Der Faden 9 ist über die Plattenoberseite O und eine Kante der Platte 1 geführt.

An einer Position Z ist das Insert 10 von Fig. 12c eingeschraubt. Zur besseren Übersicht ist dieses Insert ohne Faden dargestellt.

Figuren 14a-14h zeigen schematisch ein Verfahren zur Verwendung des Inserts 10 von Fig. 12l.

Fig. 14a zeigt zwei Fäden 9, die entlang des ersten Abschnittes der einstückigen Halterung 12,12', die zwei Abschitte umfasst, durch jeweils einen Einführschlitz seitlich an das Insert 10 herangeführt werden. Der jeweilige Einführschlitz ist zwischen den beiden Stücken der Halterung 12,12' gebildet. Die Fäden 9 können zu diesem Zeitpunkt bereits an einem Knochen gehalten und durch die Clavicula und eine Clavicula-Platte (nicht gezeigt) geführt worden sein.

Fig. 14b zeigt, wie die Fäden 9 näher an das Insert 10 geführt werden.

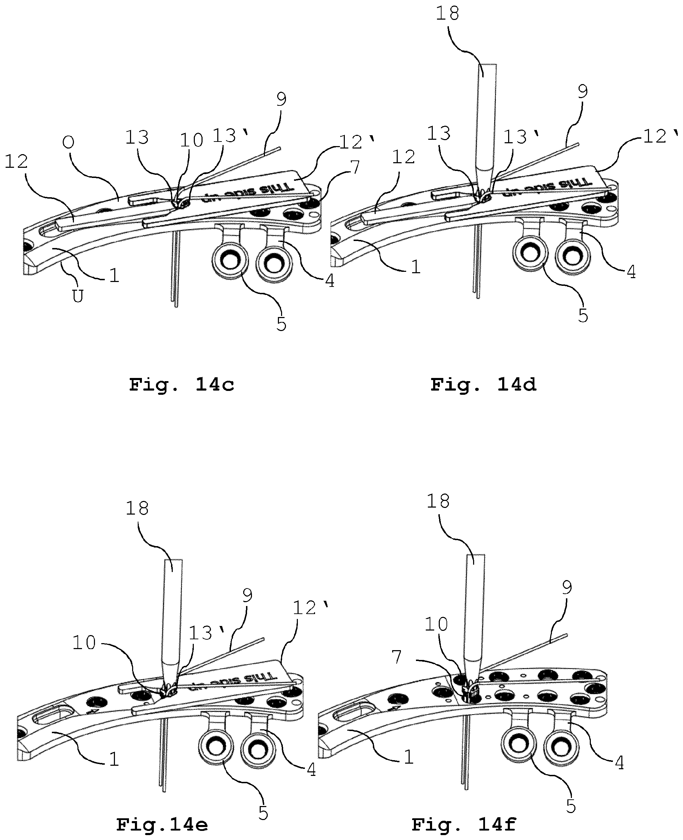

Fig. 14c zeigt, wie das Insert an die Clavicula-Platte 1 angelegt wird, so dass das Insert 10 über dem gewünschten Schraubenloch 7 positioniert ist. Die Fäden 9 werden angezogen, so dass sie im Insert 10 einrasten. Das Insert 10 ist dabei so positioniert, dass die Markierung 16 nach oben (in eine Richtung von der Plattenunterseite zur Plattenoberseite O) zeigt.

Fig. 14d zeigt, wie eine Klinge 18 eines Schraubendrehers in das Insert eingeführt wird.

Fig. 14e zeigt, wie das erste Stück 12 der Halterung an der Sollbruchstelle 13 gebrochen und entfernt wird.

Fig. 14f zeigt, wie auch das zweite Stück 12' der Halterung an der anderen Sollbruchstelle 13' abgebrochen und entfernt wird. Das Insert 10 kann in diesem Zustand mit Hilfe der Klinge 18 gehalten werden.

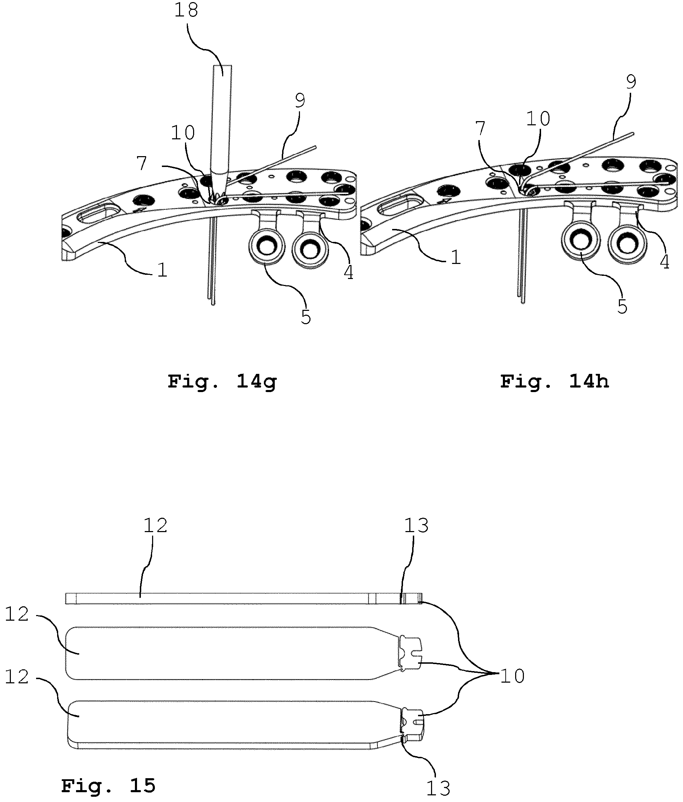

Fig. 14g zeigt, wie das Insert 10 mit Hilfe der Klinge 18 in das gewünschte Schraubenloch eingeschraubt wird.

Fig. 14h zeigt die Platte 1 mit eingeschraubtem Insert 10, wobei die Fäden 9 seitlich am Insert vorbei geführt sind. Diese könnten beispielsweise mittels eine Knotens (nicht gezeigt) über dem Insert gehalten werden.

Fig. 15 zeigt eine weitere Ausführungsform eines Inserts 10 zur Fadenfixierung in einem herkömmlichen Schraubenloch. Dieses umfasst eine Halterung 12, die über eine Sollbruchstelle 13 mit dem Insert 10 verbunden ist. Es ist so ausgebildet, dass es entlang einer Längsachse der Halterung in ein Schraubenloch eingepresst werden kann.

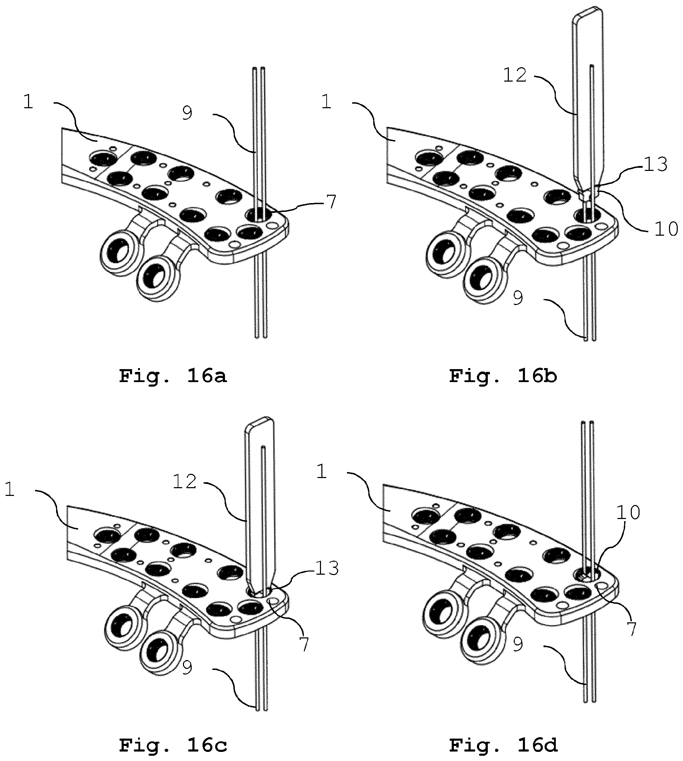

Figuren 16a-16d zeigen schematisch ein Verfahren zur Verwendung des Inserts 10 von Fig. 15.

Fig. 16a zeigt, wie in einem ersten Schritt zwei Fäden 9 durch ein Schraubenloch 7 geführt werden.

Fig. 16b zeigt, wie das Insert 10 zwischen beiden Fäden zum Schraubenloch 7 hin geführt wird.

Fig. 16c zeigt das Einpressen des Inserts 10 in das Schraubenloch 7, wodurch es rotationssicher in diesem Schraubenloch 7 fixiert ist. Beim Einpressen wird das Insert 10 zusammengedrückt und das Insert hält danach reibschlüssig im Plattenloch 7. Das hier dargestellte Stadium ist besonders geeignet, um die Halterung 12 durch Brechen der Sollbruchstelle 13 zu entfernen.

Fig. 16d zeigt die Platte 1 mit dem eingeführten Insert 10 nach Entfernen der Halterung. Die beiden Fäden 9 können durch einen Knoten über dem Insert 10 miteinander verbunden werden und umschlingen das Insert 10 daher so, dass die Fäden 9 am Insert 10 zurückgehalten werden. Gleichzeitig verhindert der Zug der Fäden, dass das Insert aus dem Plattenloch gleiten kann.

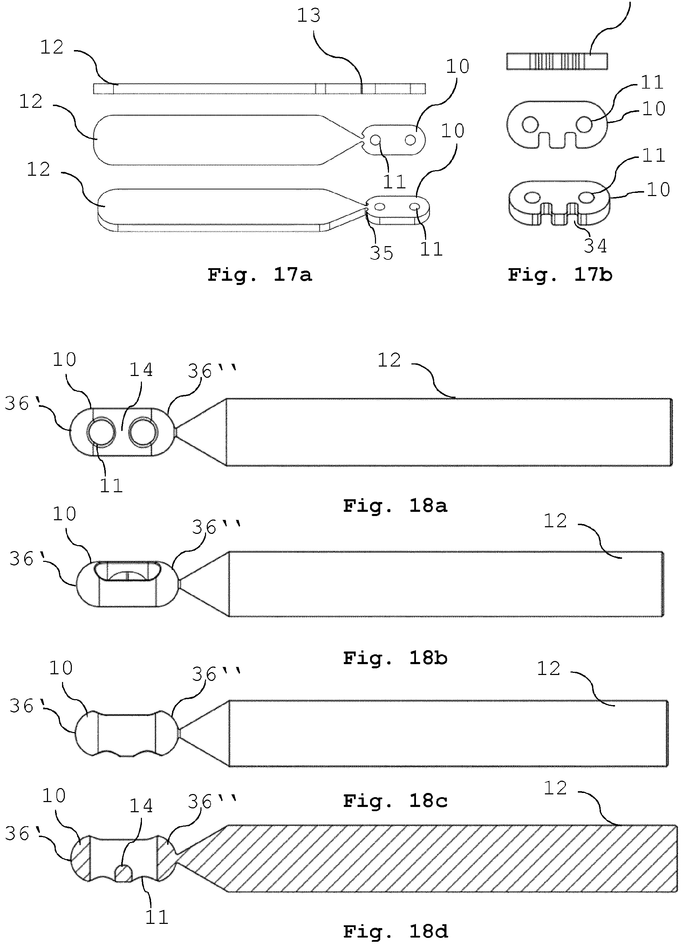

Fig. 17a zeigt eine nicht erfindungsgemässe Ausführungsform eines Inserts 10 zur Fadenfixierung in einer Seitenansicht, Draufsicht und einer perspektivischen Ansicht. Das gezeigte Insert ist zur Verwendung in einer speziell dafür ausgebildeten Aufnahme (beispielsweise 6 in Fig. 1a) angepasst. Das Insert 10 umfasst eine Halterung 12, die über eine Sollbruchstelle mit dem Insert 10 verbunden ist. Das Insert ist im Wesentlichen flach ausgeführt und hat eine teilweise gerundete Form. Das Insert 10 weist zwei Löcher 11 auf, durch die ein Faden geführt werden kann. An der Sollbruchstelle 13 hat das Insert eine Vertiefung 35 in Richtung der Löcher 11. Dadurch sind allfällig vorhandene Unregelmässigkeiten auf der Oberfläche, die vom Brechen der Sollbruchstelle 13 herrühren, vertieft und kommen nicht in Kontakt mit der Aufnahme für das Insert 10.

Fig. 17b zeigt eine weitere Variante eines Inserts 10. Dieses ist vorliegend ohne Halterung ausgeführt. Es wäre aber denkbar, dieses Insert mit einer z.B. über eine Sollbruchstelle verbundenen Halterung auszustatten. Das gezeigte Insert 10 ist ähnlich dem in Fig. 17a gezeigten. Neben der erwähnten fehlenden Halterung umfasst dieses Insert gegenüber dem in Fig. 17a gezeigten zusätzlich eine seitliche Vertiefung 34, an der das Insert mittels eines Werkzeuges (Pinzette oder anderes Halteinstrument) gegriffen werden kann.

Fig. 18a zeigt eine weitere Ausführungsform eines Inserts 10 zur Fadenfixierung in einer Unteransicht. Das Insert 10 ist ähnlich dem Insert 10 von Figuren 6a-6e. Es umfasst aber sphärische Abschnitte 36',36" an den Enden. Ein sphärischer Abschnitt 36" ist über eine Sollbruchstelle 13 mit einer Halterung verbunden. Ein Steg 14 bildet zwei Öffnungen 11, durch die ein Faden (nicht gezeigt) geführt werden kann. Der Steg 14 ist auf der Unterseite bündig mit der Oberfläche des Inserts 10. Ebenfalls weist das Insert keine Abflachung auf der Oberseite auf.

Fig. 18b zeigt das Insert 10 von Fig. 18a in einer perspektivischen Ansicht.

Fig. 18c zeigt das Insert von Fig. 18a in einer Seitenansicht.

Fig. 18d zeigt das Insert von Fig. 18a in einer Querschnittsansicht in einer Ebene parallel zu einer Längsachse des Inserts 10 und der Halterung 12. Der Steg 14 ist versenkt angeordnet und bildet zwei Öffnungen 11, durch die ein Faden 9 geführt werden kann.

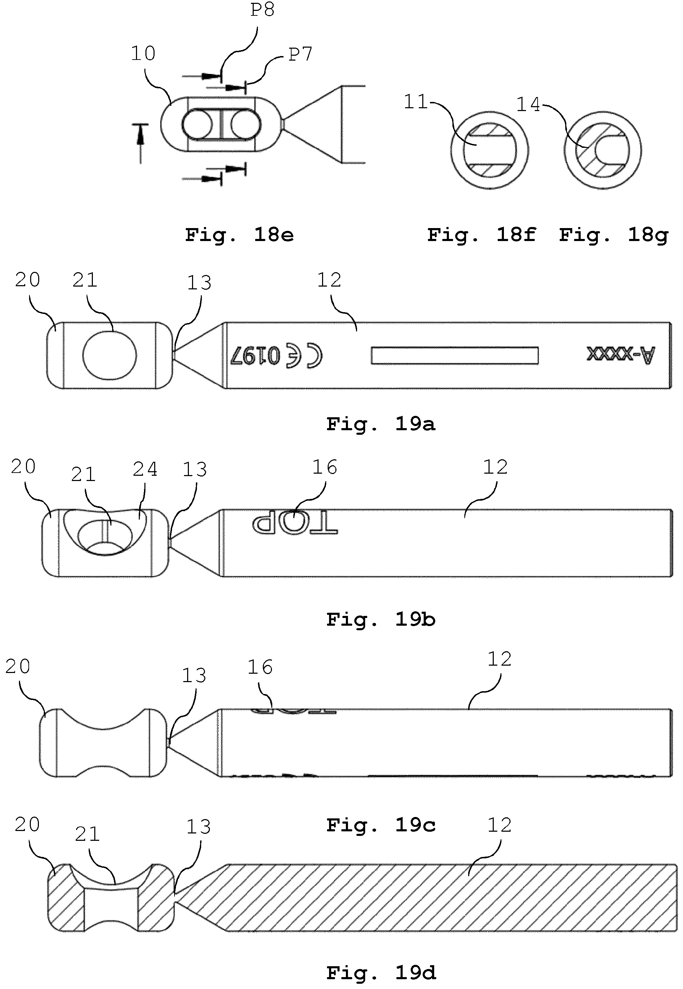

Fig. 18e zeigt eine Detailansicht des Inserts 10 von Fig. 18a.

Fig. 18f zeigt einen Querschnitt des Inserts 10 von Fig. 18a in der Ebene P7 (siehe Fig. 18e) mit einer der Öffnungen 11 zur Durchführung eines Fadens. Die Öffnung 11 ist im Wesentlichen zylinderförmig ausgeführt. Das Insert 10 hat einen im Wesentlichen kreisförmigen Querschnitt. Es wäre aber auch denkbar, das Insert 10 auf einer Seite gerade auszubilden, so dass der Querschnitt die Form eines Kreissegmentes aufweist (wie dies beispielsweise beim Insert von Figuren 6a-6e der Fall ist).

Fig. 18g zeigt einen Querschnitt des Inserts 10 von Fig. 18e in der Ebene P8, die durch den Steg 14 verläuft. Der Steg 14 ist bündig zur Aussenseite des Inserts 10 und versenkt ausgebildet.

Fig. 19a zeigt eine weitere Variante eines Inserts 20 zur Fixierung einer Schraube. Es umfasst eine Öffnung 21, die hier ohne Gewinde ausgeführt ist und die zur Aufnahme einer Schraube angepasst ist. Es ist über eine Sollbruchstelle 13 mit einer Halterung 12 verbunden.

Fig. 19b zeigt das Insert 20 von Fig. 19a in einer perspektivischen Ansicht. Das Insert 20 ist ähnlich dem von Fig. 9a-9e. Es weist allerdings gleich wie das Insert 10 von Figuren 18a-18g in einer Ebene senkrecht zu einer Längsachse der Halterung 12 und des Inserts 20 einen im Wesentlichen kreisförmigen Querschnitt auf. Ebenfalls weist das Insert keine Abflachung auf der Oberseite auf. Die Öffnung 21 ist an ihrer Oberseite mit einer Vertiefung 24 ausgebildet, so dass die Öffnung 21 als Senkloch für eine Schraube ausgebildet ist.

Fig. 19c zeigt das Insert 20 von Fig. 19a in einer Seitenansicht.

Fig. 19d zeigt das Insert 20 von Fig. 19a in einer Querschnittsansicht in einer Ebene senkrecht zur Längsachse der Halterung 12 und des Inserts 20.

Fig. 20a zeigt die Clavicula-Platte 1 von Fig. 1a in einem an einer menschlichen Clavicula B festgeschraubten Zustand. Die Platte 1 ist mit neun Schrauben 3 in Schraubenlöchern 7 fixiert. Zusätzlich sind zwei Schrauben 3 über je eine Lasche 5 am Knochen fixiert, und eine weitere Schraube 3' über ein Langloch 8. Die hier dargestellte Behandlung wurde ohne Verwendung eines Inserts 10,20 durchgeführt. Es wäre aber denkbar, ein Insert 10 in die dafür angepasste Aufnahme 6 einzuführen und einen Faden zu fixieren oder eine weitere Schraube 3 über ein Insert 20 in den Knochen B zu schrauben.

Fig. 20b zeigt die Clavicula-Platte 1 von Fig. 20a, wobei der Knochen B zur besseren Übersicht transparent dargestellt ist.

## Patentansprüche

1. Kit, umfassend mindestens ein Insert (10, 20) und eine Platte (1) zur Versorgung eines Knochens, insbesondere einer Clavicula (B), welche Platte (1) eine Aufnahme (6) für das Insert (10, 20) aufweist,
- wobei das Insert (10, 20) eine Aussenkontur (C") aufweist
- und wobei die Aufnahme (6) eine Innenkontur (C') aufweist
wobei Aussenkontur (C") und Innenkontur (C') so aufeinander abgestimmt sind, dass das in die Aufnahme (6) eingesetzte Insert (10, 20) um genau eine Achse, die im Wesentlichen parallel zu einer Plattenoberseite (O) verläuft, verschwenkbar ist, **dadurch gekennzeichnet, dass** das Kit einen Faden zum Fixieren eines Körperteils umfasst, und das Insert zur Fixierung des Fadens angepasst ist, wobei das Insert derart ausgebildet ist, dass das Insert in der Einsetzposition in der Aufnahme schwenkbar ist, wenn der Faden eingesetzt ist.

2. Kit gemäss Anspruch 1, wobei die Aussenkontur (C") des Inserts (10, 20) und/oder die Innenkontur (C') der Aufnahme (6) mindestens teilweise gerundet ist und insbesondere eine mindestens teilweise rotationssymmetrische Form, bevorzugt mindestens teilweise eine kreiszylindrische, gestuft zylindrische, konische, elliptische, hyperbolische oder parabolische Form, insbesondere in einem Querschnitt senkrecht zu einer Längsachse, aufweist.

3. Kit gemäss einem der vorangehenden Ansprüche, wobei die Aussenkontur (C") und die Innenkontur (C') so aufeinander abgestimmt sind, dass das in die Aufnahme (6) eingesetzte Insert (10, 20) in einer Längsrichtung (L) der Platte verschiebbar ist.

4. Kit gemäss einem der vorherigen Ansprüche, wobei das Insert (10) mindestens einen Steg (14), bevorzugt genau einen Steg (14), zum Halten des Fadens (9) aufweist.

5. Kit gemäss Anspruch 4, wobei der mindestens eine Steg (14) versenkt ist.

6. Kit gemäss einem der Ansprüche 1-3, wobei das Insert (20) eine Öffnung (21) zur Aufnahme einer Schraube (3) aufweist.

7. Kit gemäss einem der vorherigen Ansprüche, wobei das Insert (10, 20) eine Halterung (12) aufweist und über mindestens eine Sollbruchstelle (13) mit dieser verbunden ist.

8. Kit gemäss einem der vorherigen Ansprüche, wobei die Platte auf ihrer Oberseite mindestens eine die Platte (1) nicht durchdringende Senkung (2) aufweist, die zur temporären Aufnahme eines Werkzeuges (17), bevorzugt einer Zange, angepasst ist.

9. Kit gemäss Anspruch 8, weiter umfassend ein Werkzeug (17), bevorzugt eine Zange, wobei das Werkzeug (17) mindestens eine Spitze (32) umfasst, deren Grösse so angepasst ist, dass sie mit der Senkung (2) der Platte in Wirkverbindung gebracht werden kann.

10. Kit gemäss einem der vorangehenden Ansprüche, wobei das Insert (20) mindestens eine Öffnung (11, 21) umfasst, wobei die Öffnung (11, 21) zur Aufnahme einer Schraube (3) oder zur Fixierung des Fadens angepasst ist, und über eine Sollbruchstelle (13) mit mindestens einer Halterung (12) verbunden ist.

## Claims

1. Kit, comprising at least one insert (10, 20) and a plate (1) for treating a bone, in particular a clavicle (B), which plate (1) has a receptacle (6) for the insert (10, 20),
- wherein the insert (10, 20) has an outer contour (C")
- and wherein the receptacle (6) has an inner contour (C')
wherein the outer contour (C") and the inner contour (C') are matched to each other in such a way that the insert (10, 20) inserted into the receptacle (6) is pivotable about exactly one axis which runs substantially parallel to a plate upper side (O), **characterized in that** the kit comprises a suture for fixing a body part, and the insert is adapted for fixing the suture, wherein the insert is designed in such a way that the insert is pivotable in the insertion position in the receptacle when the suture is inserted.

2. Kit according to claim 1, wherein the outer contour (C") of the insert (10, 20) and/or the inner contour (C') of the receptacle (6) is at least partially rounded and in particular has an at least partially rotationally symmetrical shape, preferably at least partially a circular cylindrical, stepped cylindrical, conical, elliptical, hyperbolic or parabolic shape, in particular in a cross-section perpendicular to a longitudinal axis.

3. Kit according to one of the preceding claims, wherein the outer contour (C") and the inner contour (C') are matched to one another such that the insert (10, 20) inserted into the receptacle (6) is displaceable in a longitudinal direction (L) of the plate.

4. Kit according to one of the previous claims, wherein the insert (10) comprises at least one crosspiece (14), preferably exactly one crosspiece (14), for holding the suture (9).

5. Kit according to claim 4, wherein the at least one crosspiece (14) is recessed.

6. Kit according to any one of claims 1-3, wherein the insert (20) has an opening (21) for receiving a screw (3).

7. Kit according to one of the previous claims, wherein the insert (10, 20) has a holder (12) and is connected to the holder via at least one predetermined breaking point (13).

8. Kit according to one of the preceding claims, wherein the plate has on its upper side at least one recess (2) which does not extend through the plate (1) and which is adapted to receive a tool (17), preferably a pair of pliers, at a certain speed.

9. A kit according to claim 8, further comprising a tool (17), preferably a pair of pliers, said tool (17) comprising at least one tip (32) the size of which is adapted to be brought into operative engagement with the recess (2) of the plate.

10. Kit according to one of the preceding claims, wherein the insert (20) comprises at least one opening (11, 21), wherein the opening (11, 21) is adapted to receive a screw (3) or to secure the suture, and is connected to at least one holder (12) via a predetermined breaking point (13).

## Revendications

1. Kit comprenant au moins un insert (10, 20) et une plaque (1) pour le traitement d'un os, en particulier d'une clavicule (B), laquelle plaque (1) présente un logement (6) pour l'insert (10, 20),
- l'insert (10, 20) présentant un contour extérieur (C")
- et dans lequel le logement (6) présente un contour intérieur (C')
le contour extérieur (C'') et le contour intérieur (C') étant adaptés l'un à l'autre de telle sorte que l'insert (10, 20) inséré dans le logement (6) puisse pivoter autour d'exactement un axe qui s'étend sensiblement parallèlement à une face supérieure (O) de la plaque, **caractérisé en ce que** le kit comprend un fil pour fixer une partie du corps, et l'insert est adapté pour fixer le fil, l'insert étant conçu de telle sorte que l'insert peut pivoter dans la position d'insertion dans le logement lorsque le fil est inséré..

2. Kit selon la revendication 1, dans lequel le contour extérieur (C") de l'insert (10, 20) et/ou le contour intérieur (C') du logement (6) est au moins partiellement arrondi et présente en particulier une forme au moins partiellement à symétrie de révolution, de préférence au moins partiellement cylindrique circulaire, cylindrique étagée, conique, elliptique, hyperbolique ou parabolique, en particulier dans une section transversale perpendiculaire à un axe longitudinal.

3. Kit selon l'une des revendications précédentes, dans lequel le contour extérieur (C'") et le contour intérieur (C") sont adaptés l'un à l'autre de telle sorte que l'insert (10, 20) inséré dans le logement (6) peut être déplacé dans une direction longitudinale (L) de la plaque.

4. Kit selon l'une des revendications précédentes, dans lequel l'insert (10) présente au moins une traverse (14), de préférence exactement une traverse (14), pour maintenir le fil (9).

5. Kit selon la revendication 4, dans lequel ladite au moins une traverse (14) est submergée.

6. Kit selon l'une quelconque des revendications 1 à 3, dans lequel l'insert (20) présente une ouverture (21) pour recevoir une vis (3).

7. Kit selon l'une des revendications précédentes, dans lequel l'insert (10, 20) comporte un support (12) et est relié à celui-ci par au moins un point destiné à la rupture (13).

8. Kit selon l'une quelconque des revendications précédentes, dans lequel la plaque comporte sur sa face supérieure au moins un lamage (2) ne traversant pas la plaque (1) et adapté pour recevoir temporairement un outil (17), de préférence une pince.

9. Kit selon la revendication 8, comprenant en outre un outil (17), de préférence une pince, l'outil (17) comprenant au moins une pointe (32) dont la taille est adaptée pour qu'elle puisse être amenée en liaison active avec le creux (2) de la plaque.

10. Kit selon l'une quelconque des revendications précédentes, dans lequel l'insert (20) comprend au moins une ouverture (11, 21), l'ouverture (11, 21) étant adaptée pour recevoir une vis (3) ou pour fixer le fil, et étant reliée à au moins un support (12) par l'intermédiaire d'un point de rupture (13).
